# EUROPEAN PATENT SPECIFICATION

(11) **EP 2 904 397 B1**
(45) Date of publication and mention of the grant of the patent: **18.07.2018**
(21) Application number: 13771159.4
(22) Date of filing: 02.10.2013
(51) Int. Cl.: G01N 33/574, G01N 33/483, G06F 19/12, G16H 50/20

(54) **MEANS AND METHODS FOR DIAGNOSING RECURRENCE OF PROSTATE CANCER AFTER PROSTATECTOMY**
MITTEL UND VERFAHREN ZUR DIAGNOSE DER WIEDERERKRANKUNG AN PROSTATAKREBS NACH EINER PROSTATEKTOMIE
MOYENS ET MÉTHODES PERMETTANT DE DIAGNOSTIQUER LA RÉCIDIVE D'UN CANCER DE LA PROSTATE APRÈS UNE PROSTATECTOMIE

(30) Priority: 02.10.2012 EP 12187029; 02.10.2012 US 201261708686 P; 02.07.2013 EP 13174777
(43) Date of publication of application: 12.08.2015
(73) Proprietor: metanomics Health GmbH, 10589 Berlin (DE); Charite - Universitätsmedizin Berlin, 10117 Berlin (DE)
(72) Inventor: RESZKA, Regina, 16341 Panketal (DE); KAMLAGE, Beate, 12161 Berlin (DE); BETHAN, Bianca, 10717 Berlin (DE); JUNG, Klaus, 10409 Berlin (DE); LEIN, Michael, 14532 Stahnsdorf (DE); KRISTIANSEN, Glen, 53127 Bonn (DE); STEPHAN, Carsten, 10367 Berlin (DE)
(74) Representative: Herzog, Fiesser & Partner Patentanwälte PartG mbB
(86) International application number: PCT/EP2013/070576
(87) International publication number: WO 2014/053564

(56) References cited:
- EP-A1- 1 553 414
- WO-A1-2010/139711
- US-A1- 2008 181 850
- GONZALEZ C M ET AL: "Preoperative PSA level significantly associated with interval to biochemical progression after radical retropubic prostatectomy", UROLOGY, BELLE MEAD, NJ, US, vol. 64, no. 4, 1 October 2004 (2004-10-01), pages 723-728, XP004604388, ISSN: 0090-4295, DOI: 10.1016/J.UROLOGY.2004.05.019
- S. A. TAHIR: "Preoperative Serum Caveolin-1 as a Prognostic Marker for Recurrence in a Radical Prostatectomy Cohort", CLINICAL CANCER RESEARCH, vol. 12, no. 16, 15 August 2006 (2006-08-15), pages 4872-4875, XP055088283, ISSN: 1078-0432, DOI: 10.1158/1078-0432.CCR-06-0417

## Description

The present invention concerns means and methods for cancer diagnosis and, in particular, diagnosis of prostate cancer. Specifically, it relates to a method for diagnosing whether a subject is at risk for recurrent prostate carcinoma after prostatectomy comprising determining the amount of at least one metabolite selected from Table 2 in a test sample of a subject after prostatectomy, and comparing the determined amount to a reference, whereby it is diagnosed whether the subject is at risk for recurrent prostate carcinoma after prostatectomy. Further methods provided in accordance with the present invention relate to differentiating between a malignant prostate carcinoma tissue and a non-malignant tissue and diagnosing metastasizing prostate carcinoma in a subject. Also provided are devices for carrying out the aforementioned methods.

Prostate cancer (PCA) is an uncontrolled (malignant) growth of cells in the prostate gland and the most common malignancy in the western world. The cause of prostate cancer isn't fully understood at present. More than 180,000 new cases of PCA patients were diagnosed 2008 in the U.S. The expected prevalence for the US will be 2,175,699 prostate cancer patients in 2010. There are several tests used to diagnose prostate cancer which include Digital rectal examination (DRE), Transrectal ultrasound (TRUS), Prostate-specific antigen (PSA) free and total,, PSA derivatives as percent free PSA (%fPSA), age-specific PSA values, PSA density, PSA velocity, Prostatic acid phosphatase (PAP) test, Prostate biopsy, Computed tomography (CT scan), Bone scan and/or MRI.

The prostate-specific antigen (PSA) plasma test has been a major factor in increasing awareness and better patient management of PCA, but its lack of specificity limits its use in diagnosis and makes it suitable for early detection of PCA. PSA is organ but not cancer specific. This leads to a high number of false-positive findings. The diagnosis of PCA can be confirmed only by a biopsy. More than 800,000 of this invasive and costly procedure were done every year in the U.S. although controversies over PCA screening persist (Wilson 2004, Clin. Prostate Cancer 3: 21-25, Crawford 2005, Lancet 365: 1447-1449). The early detection of PCA is likely (Schröder 2009; N Engl J Med. Mar 26;360(13):1320-1328; Andriole 2009, N Engl J Med Mar 26;360(13):1310-1319

The use of metabolomics to generate biomarkers for cancer diagnosis or prognosis and therapeutic evaluation is currently in focus (Spratlin 2009, Clin Cancer Res 15(2): 431-440). Others had published absolute concentrations of the metabolites citrate, myo-inositol and spermine in human expressed prostatic secretions (EPS) as age-independent markers of PCA (Serkova 2008, The Prostate 68: 620-628). Recently, metabolomic profiles from tissue plasma and post-digital-rectal-exam urine samples of biopsy-positive PCA patients and biopsy-negative control individuals were assayed (Sreekumar 2009, Nature 457(12): 910-914). Sarcosine was suggested as one potential biomarker for prostate cancer progression. Metabolite profiling revealed further metabolites for diagnosing and staging of prostate cancer (WO2010/139711 A1).

However, metabolic biomarkers providing differential or additional diagnostic information are still highly desired. For example, health and therapeutic management of patients would benefit from identifying patients which are at risk of developing recurrent prostate cancer after prostatectomy. Moreover, the identification of metastasizing prostate cancers would also be beneficial for clinical care of the patients. Finally, in light of the high number of false-positive or false-negative diagnosis of prostate carcinoma, there is still a high need for biomarkers which allow for a reliable and efficient diagnosis or prognosis of prostate carcinoma.

The technical problem underlying the present invention can be seen as the provision of means and methods for complying with the aforementioned needs. The technical problem is solved by the embodiments characterized in the claims and herein below.

Accordingly, the present invention relates to a method for diagnosing whether a subject is at risk for recurrent prostate carcinoma after prostatectomy comprising:
(a) determining the amount of at least one metabolite selected from Table 2 in a test sample of a subject after prostatectomy; and
(b) comparing the amount determined in step (a) to a reference, whereby it is diagnosed whether the subject is at risk for recurrent prostate carcinoma after prostatectomy.

The aforementioned method and the other methods referred to herein may comprise steps in addition to those explicitly mentioned. Such steps may be steps of sample pre-treatment or data evaluation as also specified elsewhere herein in detail. Moreover, the methods may be assisted by automation. For example, the determination of the amount referred to in step a) may be carried out by a detector device which is supplied with samples by a suitable robotic device. The comparison may be carried out by a suitable algorithm implemented on a data processor such as a computer and could, thus, be carried out in a computer-implemented manner.

The term "diagnosing" as used herein refers to assessing the probability according to which a subject is suffering from a disease or condition or is at risk of developing a disease or condition. Accordingly, the method provides an aid for diagnosis since it might be necessary to further strengthen or confirm said diagnosis by, e.g., a medical practitioner. In particular, as will be understood by those skilled in the art, such an assessment, although preferred to be, may usually not be correct for 100% of the subjects to be diagnosed. The term, however, requires that a statistically significant portion of subjects can be identified as suffering from the disease or as having a predisposition therefor. Whether a portion is statistically significant can be determined without further ado by the person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 50%, at least 60%, at least 70%, at least 80%, at least 90%, at least 95%. The p-values are, preferably, 0.2, 0.1, 0.05. It will be understood, moreover, that the methods of the present invention essentially provide an aid for diagnosis and may be included into or supplemented by other diagnostic measures. Diagnosing according to the present invention includes monitoring, confirmation, and classification of the relevant disease or its symptoms. Monitoring relates to keeping track of an already diagnosed disease, or a complication, e.g. to analyze the progression or regression of the disease, the influence of a particular treatment on the progression of disease or complications arising during the disease period or after successful treatment of the disease. Confirmation relates to the strengthening or substantiating a diagnosis already performed using other indicators or markers. Classification relates to allocating the diagnosis according to the strength or kind of symptoms into different classes, e.g. the stages for prostate carcinomas as set forth elsewhere in the description. Being at risk as used herein means that a subject has not yet developed the disease or condition but, nevertheless, will develop it in the future with a certain likelihood. Said likelihood shall differ significantly from the likelihood of statistical appearance of the disease or condition. Preferably, the likelihood for developing a prostate carcinoma is at least 30%, at least 40%, at least 50%, at least 60%, at least 70%, at least 80%, at least 90% or 100% of a predisposition is diagnosed. Diagnosis of a predisposition may sometimes be referred to as prediction of the likelihood that a subject will develop the disease.

The term "prostate carcinoma" as used herein refers to a malignant tumor developed from prostate gland cells. Said cells may metastasize from the prostate to other tissues or organs, especially to the bones and lymph nodes. Early prostate carcinomas usually cause no symptoms. Prostate carcinomas in advanced stages may cause pain, difficulty in urinating, problems during sexual intercourse, and erectile dysfunction. Further symptoms of prostate carcinomas are well known in the art including frequent urination, increased urination at night, difficulty starting and maintaining a steady stream of urine, blood in the urine, and painful urination and are described in the standard text books of medicine, such as Stedman or Pschyrembl. The Gleason scoring system is used to grade prostate tumors from 2 to 10, whereby a Gleason score of 10 indicates the most abnormalities.

The term "at least one metabolite" as used herein refers to a single metabolite or to a plurality of metabolites, i.e. preferably at least 2, 3, 4, 5, 10, 50, 100, 500, 1,000, 2,000, 3,000, 5,000 or 10,000 metabolites. It is to be understood that "metabolite" as used herein may be at least one molecule of said metabolite up to a plurality of molecules of the metabolite and that a plurality of metabolites means a plurality of chemically different molecules wherein for each metabolite at least one molecule up to a plurality of molecules may be present. A metabolite in accordance with the present invention encompasses all classes of organic or inorganic chemical compounds including those being comprised by biological material such as organisms. Preferably, the metabolite in accordance with the present invention is a small molecule compound. More preferably, in case a plurality of metabolites is envisaged, said plurality of metabolites representing a metabolome, i.e. the collection of metabolites being comprised by an organism, an organ, a tissue or a cell at a specific time and under specific conditions. Each of the metabolites from the Tables below is a suitable biomarker by its own for the diseases referred to herein. However, most preferably, a group of biomarkers is to be determined by the method of the present invention. A group of biomarkers comprises or consists, preferably, of at least two, at least three, at least four and, preferably, up to all of the aforementioned biomarkers.

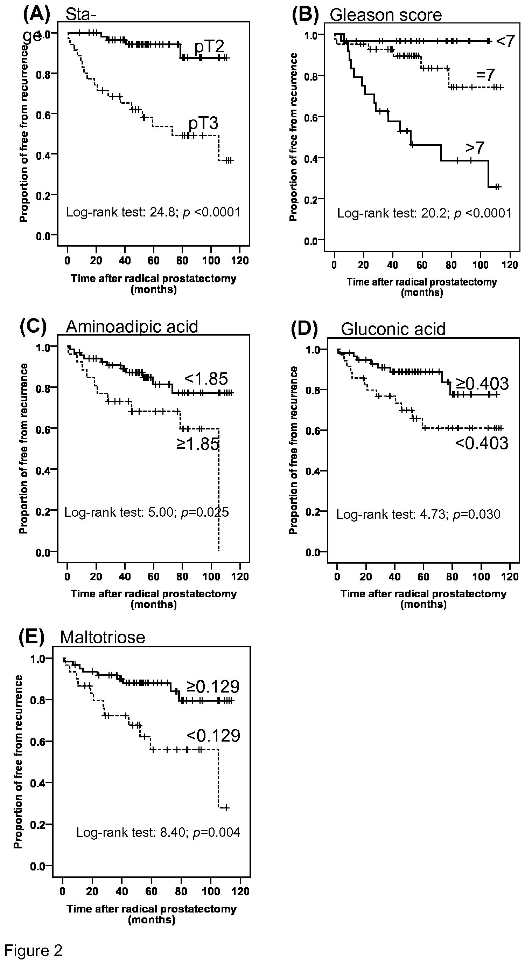

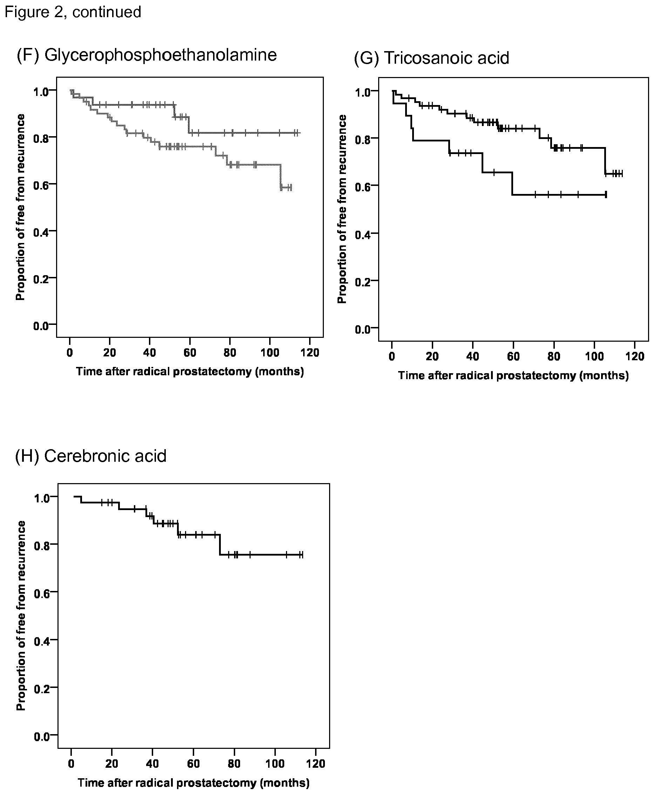

Particularly preferred as at least one metabolite to be determined as a biomarker according to the present invention is aminoadipic acid, gluconic acid, or maltotriose. Moreover also preferably as at least one metabolite to be determined as a biomarker according to the present invention is tricosanoic acid, glycerophosphoethanolamine and Cerebronic acid (see Figure 2).

The metabolites are small molecule compounds, such as substrates for enzymes of metabolic pathways, intermediates of such pathways or the products obtained by a metabolic pathway. Metabolic pathways are well known in the art and may vary between species. Preferably, said pathways include at least citric acid cycle, respiratory chain, photosynthesis, photorespiration, glycolysis, gluconeogenesis, hexose monophosphate pathway, oxidative pentose phosphate pathway, production and β-oxidation of fatty acids, urea cycle, amino acid biosynthesis pathways, protein degradation pathways such as proteasomal degradation, amino acid degrading pathways, biosynthesis or degradation of: lipids, polyketides (including e.g. flavonoids and iso-flavonoids), isoprenoids (including eg. terpenes, sterols, steroids, carotenoids, xanthophylls), carbohydrates, phenylpropanoids and derivatives, alcaloids, benzenoids, indoles, indole-sulfur compounds, porphyrines, anthocyans, hormones, vitamins, cofactors such as prosthetic groups or electron carriers, lignin, glucosinolates, purines, pyrimidines, nucleosides, nucleotides and related molecules such as tRNAs, microRNAs (miRNA) or mRNAs. Accordingly, small molecule compound metabolites are preferably composed of the following classes of compounds: alcohols, alkanes, alkenes, alkines, aromatic compounds, ketones, aldehydes, carboxylic acids, esters, amines, imines, amides, cyanides, amino acids, peptides, thiols, thioesters, phosphate esters, sulfate esters, thioethers, sulfoxides, ethers, or combinations or derivatives of the aforementioned compounds. The small molecules among the metabolites may be primary metabolites which are required for normal cellular function, organ function or animal growth, development or health. Moreover, small molecule metabolites further comprise secondary metabolites having essential ecological function, e.g. metabolites which allow an organism to adapt to its environment. Furthermore, metabolites are not limited to said primary and secondary metabolites and further encompass artificial small molecule compounds. Said artificial small molecule compounds are derived from exogenously provided small molecules which are administered or taken up by an organism but are not primary or secondary metabolites as defined above. For instance, artificial small molecule compounds may be metabolic products obtained from drugs by metabolic pathways of the animal. Moreover, metabolites further include peptides, oligopeptides, polypeptides, oligonucleotides and polynucleotides, such as RNA or DNA. More preferably, a metabolite has a molecular weight of 50 Da (Dalton) to 30,000 Da, most preferably less than 30,000 Da, less than 20,000 Da, less than 15,000 Da, less than 10,000 Da, less than 8,000 Da, less than 7,000 Da, less than 6,000 Da, less than 5,000 Da, less than 4,000 Da, less than 3,000 Da, less than 2,000 Da, less than 1,000 Da, less than 500 Da, less than 300 Da, less than 200 Da, less than 100 Da. Preferably, a metabolite has, however, a molecular weight of at least 50 Da. Most preferably, a metabolite in accordance with the present invention has a molecular weight of 50 Da up to 1,500 Da.

It will be understood that in addition to the aforementioned metabolites or groups of metabolites, an additional biomarker or a group of additional biomarkers can be determined by the method of the present invention as well. Said additional biomarkers include nucleic acids, peptides, polypeptides or other clinical parameters known to be associated with prostate carcinomas or predisposition therefore. Preferably, said additional biomarker is selected from the group consisting of: Digital rectal examination (DRE), Transrectal ultrasound (TRUS), PSA and PAP Tests, Prostate-specific antigen (PSA), Free and total PSA (also known as PSA II), Age-specific PSA, Prostatic acid phosphatase (PAP) test Tumor Biopsy, Computed tomography (CT scan), Bone scan and MRI.

The supportive metabolites referred to before will, preferably, also be compared to suitable reference results as specified elsewhere herein. The result of the said comparison will be further supportive for the finding as to whether the subject will suffer from prostate carcinomas or not or will have a predisposition therefor or not. Preferred reference results, values for changes of the relative amounts and indications for the kind of regulation are to be found in the accompanying Examples, below.

The term "test sample" as used herein refers to samples to be used for the diagnosis of prostate carcinomas or a predisposition therefor by the method of the present invention. Said test sample is a biological sample. Samples from biological sources (i.e. biological samples) usually comprise a plurality of metabolites. Preferred biological samples to be used in the method of the present invention are samples from body fluids, preferably, blood, plasma, serum, lymph, or urine, or samples derived, e.g., by biopsy, from cells, tissues or organs, preferably prostate tissue suspected to include or essentially consist of prostate carcinoma cells. This also encompasses samples comprising subcellular compartments or organelles, such as the mitochondria, Golgi network or peroxisomes. Biological samples can be derived from a subject as specified elsewhere herein. Techniques for obtaining the aforementioned different types of biological samples are well known in the art. For example, blood samples may be obtained by blood taking while tissue or organ samples are to be obtained, e.g., by biopsy.

The aforementioned samples are, preferably, pre-treated before they are used for the method of the present invention. As described in more detail below, said pre-treatment may include treatments required to release or separate the compounds or to remove excessive material or waste. Suitable techniques comprise centrifugation, extraction, fractioning, purification and/or enrichment of compounds. Moreover, other pre-treatments are carried out in order to provide the compounds in a form or concentration suitable for compound analysis. In particular, the samples may be pre-treated by anticoagulation agents such as EDTA-, ammonium or lithium heparinate-, acid-citrate-dextrose (ACD)-, oxalate- or citrate- buffers if the sample is a blood plasma sample or with clotting activators if the sample is a serum sample. Tissue samples may be pre-treated by paraffin embedding, or by fixation agents or measures such as liquid nitrogen, freeze-drying or formaldehyde solutions. Moreover, for example, if gas-chromatography coupled mass spectrometry is used in the method of the present invention, it will be required to derivatize the compounds prior to the said gas chromatography. Suitable and necessary pre-treatments depend on the means used for carrying out the method of the invention and are well known to the person skilled in the art. Pre-treated samples as described before are also comprised by the term "sample" as used in accordance with the present invention.

The term "subject" as used herein relates to animals, preferably to mammals such as mice, rats, sheep, dogs, cats, horses, monkeys, or cows and, also preferably, to humans. It is to be understood that the subject to be tested comprises prostate tissue. Thus, the subject shall be a male subject, in particular a male human. Other animals which may be diagnosed applying the method of the present invention are birds or reptiles. Preferably, the subject has been subjected to prostatectomy prior to carrying out the aforementioned method of the invention. After prostatectomy, the subject shall, preferably, not show symptoms or clinical signs indicative for prostate carcinomas.

The term "determining said at least one metabolite" as used herein refers to determining at least one characteristic feature of the at least one metabolite comprised by the sample referred to herein. Characteristic features in accordance with the present invention are features which characterize the physical and/or chemical properties including biochemical properties of a metabolite. Such properties include, e.g., molecular weight, viscosity, density, electrical charge, spin, optical activity, colour, fluorescence, chemoluminescence, elementary composition, chemical structure, capability to react with other compounds, capability to elicit a response in a biological read out system (e.g., induction of a reporter gene) and the like. Values for said properties may serve as characteristic features and can be determined by techniques well known in the art. Moreover, the characteristic feature may be any feature which is derived from the values of the physical and/or chemical properties of a metabolite by standard operations, e.g., mathematical calculations such as multiplication, division or logarithmic calculus. Most preferably, the at least one characteristic feature allows the determination and/or chemical identification of the said at least one metabolite.

The at least one metabolite comprised by a test sample may be determined in accordance with the present invention quantitatively or qualitatively. For qualitative determination, the presence or absence of the metabolite will be determined by a suitable technique. Moreover, qualitative determination may, preferably, include determination of the chemical structure or composition of the metabolite. For quantitative determination, either the precise amount of the at least one metabolite present in the sample will be determined or the relative amount of the at least one metabolite will be determined, preferably, based on the value determined for the characteristic feature(s) referred to herein above. The relative amount may be determined in a case were the precise amount of a metabolite can or shall not be determined. In said case, it can be determined whether the amount in which the metabolite is present is enlarged or diminished with respect to a second sample comprising said metabolite in a second amount. Quantitatively analysing a metabolite, thus, also includes what is sometimes referred to as semi-quantitative analysis of a metabolite.

Moreover, determining as used in the method according to the present invention, preferably, includes using a compound separation step prior to the analysis step referred to before. Preferably, said compound separation step yields a time resolved separation of the metabolites comprised by the sample. Suitable techniques for separation to be used preferably in accordance with the present invention, therefore, include all chromatographic separation techniques such as liquid chromatography (LC), high performance liquid chromatography (HPLC), gas chromatography (GC), thin layer chromatography, size exclusion or affinity chromatography. These techniques are well known in the art and can be applied by the person skilled in the art without further ado. Most preferably, LC and/or GC are chromatographic techniques to be envisaged by the method of the present invention. Suitable devices for such determination of metabolites are well known in the art. Preferably, mass spectrometry is used in particular gas chromatography mass spectrometry (GC-MS), liquid chromatography mass spectrometry (LC-MS), direct infusion mass spectrometry or Fourier transform ion-cyclotrone-resonance mass spectrometry (FT-ICR-MS), capillary electrophoresis mass spectrometry (CE-MS), high-performance liquid chromatography coupled mass spectrometry (HPLC-MS), quadrupole mass spectrometry, any sequentially coupled mass spectrometry, such as MS-MS or MS-MS-MS, inductively coupled plasma mass spectrometry (ICP-MS), pyrolysis mass spectrometry (Py-MS), ion mobility mass spectrometry or time of flight mass spectrometry (TOF). Most preferably, LC-MS and/or GC-MS are used as described in detail below. Said techniques are disclosed in, e.g., Niessen, Journal of Chromatography A, 703, 1995: 37-57, US 4,540,884 or US 5,397,894, the disclosure content of which is hereby incorporated by reference. As an alternative or in addition to mass spectrometry techniques, the following techniques may be used for compound determination: nuclear magnetic resonance (NMR), magnetic resonance imaging (MRI), Fourier transform infrared analysis (FT-IR), ultra violet (UV) spectroscopy, refraction index (RI), fluorescent detection, radiochemical detection, electrochemical detection, light scattering (LS), dispersive Raman spectroscopy or flame ionisation detection (FID). These techniques are well known to the person skilled in the art and can be applied without further ado. The method of the present invention shall be, preferably, assisted by automation. For example, sample processing or pre-treatment can be automated by robotics. Data processing and comparison is, preferably, assisted by suitable computer programs and databases. Automation as described herein before allows using the method of the present invention in high-throughput approaches.

Moreover, the at least one metabolite can also be determined by a specific chemical or biological assay. Said assay shall comprise means which allow to specifically detect the at least one metabolite in the sample. Preferably, said means are capable of specifically recognizing the chemical structure of the metabolite or are capable of specifically identifying the metabolite based on its capability to react with other compounds or its capability to elicit a response in a biological read out system (e.g., induction of a reporter gene). Means which are capable of specifically recognizing the chemical structure of a metabolite are, preferably, antibodies or other proteins which specifically interact with chemical structures, such as receptors or enzymes. Specific antibodies, for instance, may be obtained using the metabolite as antigen by methods well known in the art. Antibodies as referred to herein include both polyclonal and monoclonal antibodies, as well as fragments thereof, such as Fv, Fab and F(ab)₂ fragments that are capable of binding the antigen or hapten. The present invention also includes humanized hybrid antibodies wherein amino acid sequences of a non-human donor antibody exhibiting a desired antigen-specificity are combined with sequences of a human acceptor antibody. Moreover, encompassed are single chain antibodies. The donor sequences will usually include at least the antigen-binding amino acid residues of the donor but may comprise other structurally and/or functionally relevant amino acid residues of the donor antibody as well. Such hybrids can be prepared by several methods well known in the art. Suitable proteins which are capable of specifically recognizing the metabolite are, preferably, enzymes which are involved in the metabolic conversion of the said metabolite. Said enzymes may either use the metabolite as a substrate or may convert a substrate into the metabolite. Moreover, said antibodies may be used as a basis to generate oligopeptides which specifically recognize the metabolite. These oligopeptides shall, for example, comprise the enzyme's binding domains or pockets for the said metabolite. Suitable antibody and/or enzyme based assays may be RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA) or solid phase immune tests. Moreover, the metabolite may also be identified based on its capability to react with other compounds, i.e. by a specific chemical reaction. Suitable reactions are well known in the art and, preferably encompass enzymatic reactions (e.g for mannose Pitkanen E, Pitkanen O, Uotila L.; Eur J Clin Chem Clin Biochem. 1997 Oct;35(10):761-6; or ascorbic acid Winnie Lee, Susan M. Roberts and Robert F. Labbe; Clinical Chemistry 43: 154-157, 1997), enzymatic spectrophotometric methods (BN La Du, RR Howell, PJ Michael and EK Sober; Pediatrics, Jan 1963, 39-46, Vol 31, No. 1), spectrofluorimetric methods (Sumi T, Umeda Y, Kishi Y, Takahashi K, Kakimoto F.; Clin Chim Acta. 1976 Dec 1;73(2):233-9) and fluorescence; chemiluminescence (J.J. Thiele, H.J. Freisleben, J. Fuchs and F.R. Ochsendorf; Human Reproduction, Vol. 10, No. 1, pp. 110-115, 1995). Further detection methods such as capillary electrophoresis (Hubert A. Carchon and Jaak Jaeken; 47: 1319-1321, 2001) and colorimetric methods (Kyaw A; Clin Chim Acta. 1978 Jun;86(2):153-7) can be used. Further, the metabolite may be determined in a sample due to its capability to elicit a response in a biological read out system. The biological response shall be detected as read out indicating the presence and/or the amount of the metabolite comprised by the sample. The biological response may be, e.g., the induction of gene expression or a phenotypic response of a cell or an organism.

Further, it is to be understood that depending of the technique used for determining the said at least one metabolite, the analyte which will be detected could be a derivative of the physiologically occurring metabolite, i.e. the metabolite present within a subject. Such analytes may be generated as a result of sample preparation or detection means. The compounds referred to herein are deemed to be analytes. However, as set forth above, these analytes will represent the metabolites in a qualitative and quantitative manner. Moreover, it is to be understood that for a plurality of metabolites, the metabolite will be identical to the analyte.
A metabolite or analyte as referred to in accordance with the present invention refers to a molecular species which serves as an indicator for a disease or effect as referred to in this specification. Said molecular species can be a metabolite itself which is found in a sample of a subject. Moreover, the biomarker may also be a molecular species which is derived from said metabolite. In such a case, the actual metabolite will be chemically modified in the sample or during the determination process and, as a result of said modification, a chemically different molecular species, i.e. the analyte, will be the determined molecular species. It is to be understood that in such a case, the analyte represents the actual metabolite and has the same potential as an indicator for the respective medical condition. Moreover, a biomarker according to the present invention is not necessarily corresponding to one molecular species. Rather, the biomarker may comprise stereoisomers or enantiomers of a compound. Further, a biomarker can also represent the sum of isomers of a biological class of isomeric molecules. Said isomers shall exhibit identical analytical characteristics in some cases and are, therefore, not distinguishable by various analytical methods including those applied in the accompanying Examples described below. However, the isomers will share at least identical sum formula parameters and, thus, in the case of, e.g., lipids an identical chain length and identical numbers of double bonds in the fatty acid and/or sphingobase moieties.

The term "reference" refers to amounts or values representing them, i.e. data of characteristic features of the at least one metabolite, which can be correlated to the presence or absence of a disease or condition referred to herein. Such a reference is, preferably, obtained from a sample of a subject or group of subjects known to be at risk for recurrence of prostate carcinoma after prostatectomy. The reference can, e.g., be the average or mean obtained from a group of such samples. If the reference shall be obtained from one or more biopsy tissue sample(s), the said sample(s) shall comprise or essentially consists of prostate carcinoma tissue or tissue suspected to be prostate carcinoma tissue. The latter tissue suspected to be prostate carcinoma tissue may appear as normal prostate carcinoma tissue histologically but may be derived from areas positioned in proximity to the former prostate carcinoma which was removed by prostatectomy. The reference may be obtained by applying the method of the present invention.

Alternatively, but nevertheless also preferred, the reference results may be obtained from sample of a subject or a group of subjects known not to be at risk of recurrence of prostate carcinoma after prostatectomy. Likewise, if the reference shall be obtained from a biopsy tissue sample, the said sample shall essentially consist of apparently healthy prostate tissue. The reference can also be the average or mean obtained from a group of such samples. Preferably, if biopsy tissues samples are envisaged, the sample underlying the reference and the test sample can be obtained from the same subject, i.e. from areas which are apparently affected by prostate carcinoma and from areas suspected to be affected by prostate carcinoma.

Moreover, the reference, also preferably, could be a calculated reference, most preferably the average or median, for the relative or absolute amount of a metabolite of a representative population of individuals which are apparently healthy or suffer from prostate carcinoma, wherein the subjects suffering from prostate carcinoma are within the prevalence for the disease in a given population, preferably, the US, Asian or European population The absolute or relative amounts of the metabolites of said individuals of the population can be determined as specified elsewhere herein. How to calculate a suitable reference value, preferably, the average or median, is well known in the art. The population of subjects referred to before shall comprise a plurality of subjects, preferably, at least 5, 10, 50, 100, 1,000 or 10,000 subjects. It is to be understood that the subject to be diagnosed by the method of the present invention and the subjects of the said plurality of subjects are of the same species.

More preferably, a "reference" will be obtained by determining the values for the at least one characteristic feature for a group of reference subjects, i.e. a group of subjects known to suffer from prostate carcinoma, a group of subjects known not to suffer from prostate carcinoma, a population comprising the subject to be investigated or a group of tissue biopsy samples of prostate carcinoma tissue or apparently healthy tissue and calculating the reference by appropriate statistic measures including those referred to elsewhere herein, such as median, average, quantiles, PLS-DA, logistic regression methods, random forest classification or others that give a threshold value. The threshold value should take the desired clinical settings of sensitivity and specificity of the diagnostic and prognostic test into consideration. Threshold amounts to be used as references may be, preferably, determined by applying receiver-operating characteristics (ROC) (see especially Zweig 1993, Clin. Chem. 39:561-577). The ROC graph is a plot of all of the sensitivity/specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects being at increased risk for mortality or those which have a normal risk among a cohort of subjects suffering from acute inflammation can be generated, preferably, by establishing a ROC for said cohort as described above and deriving a threshold amount therefrom. Dependent on a desired sensitivity and specificity for a diagnostic method, the ROC plot allows deriving suitable thresholds.

More preferably, the reference results, i.e. values for at least one characteristic features of the at least one metabolite, will be stored in a suitable data storage medium such as a database and are, thus, also available for future diagnoses. This also allows efficiently diagnosing predisposition for a disease or a condition because suitable reference results can be identified in the database once it has been confirmed (in the future) that the subject from which the corresponding reference sample was obtained (indeed) developed prostate carcinoma. Preferred reference results which are associated with prostate carcinoma or predisposition therefore in humans are those shown in the Tables of the accompanying Examples.

The term "comparing" refers to assessing whether the results of the determination described hereinabove in detail, i.e. the results of the qualitative or quantitative determination of the at least one metabolite, are identical or similar to reference results or differ therefrom.

In case the reference results are obtained from a subject or a group of subjects known to show the disease or condition, the said disease or condition can be diagnosed based on the degree of identity or similarity between the test amount obtained from the test sample and the aforementioned reference, i.e. based on an identical or similar qualitative or quantitative composition with respect to the at least one metabolite. The results of the test sample and the reference results are identical, if the values for the characteristic features and, in the case of quantitative determination, the intensity values are identical. Said results are similar, if the values of the characteristic features are identical but the intensity values are different. Such a difference is, preferably, not significant and shall be characterized in that the values for the intensity are within at least the interval between 1^{st} and 99^{th} percentile, 5^{th} and 95^{th} percentile, 10^{th} and 90^{th} percentile, 20^{th} and 80^{th} percentile, 30^{th} and 70^{th} percentile, 40^{th} and 60^{th} percentile of the reference value or the 50^{th}, 60^{th}, 70^{th}, 80^{th}, 90^{th} or 95^{th} percentile of the reference value.

In case the reference results are obtained from a subject or a group of subjects known not to show the disease or condition, the said disease or condition can be diagnosed based on the differences between the test amount obtained from the test sample and the aforementioned reference, i.e. differences in the qualitative or quantitative composition with respect to the at least one metabolite. The same applies if a calculated reference as specified above is used. The difference may be an increase in the absolute or relative amount of a metabolite (sometimes referred to as up-regulation of the metabolite; see also Tables, below) or a decrease in either of said amounts or the absence of a detectable amount of the metabolite (sometimes referred to as down-regulation of the metabolite; see also Tables, below). Preferably, the difference in the relative or absolute amount is significant, i.e. outside of the interval between 45^{th} and 55^{th} percentile, 40^{th} and 60^{th} percentile, 30^{th} and 70^{th} percentile, 20^{th} and 80^{th} percentile, 10^{th} and 90^{th} percentile, 5^{th} and 95^{th} percentile, 1^{st} and 99^{th} percentile of the reference value.

For the specific metabolites referred to in this specification elsewhere, preferred values for the changes in the relative amounts (i.e. changes in the median) or the kind of regulation (i.e. "up"- or "down"-regulation resulting in a higher or lower relative and/or absolute amount) are indicated in the Tables, below. If it is indicated in said Tables that a given metabolite is "up- regulated" in a subject or a tissue sample, the relative and/or absolute amount will be increased, if it is "downregulated", the relative and/or absolute amount of the metabolite will be decreased. Moreover, the Median indicates the degree of increase or decrease, e.g., a Median of 2.0 means that the amount is twice the amount of the metabolite compared to the reference.

In a preferred embodiment of the aforementioned method of the invention, said reference is derived from a subject or group of subjects known to be at risk for recurrent prostate carcinoma after prostatectomy. More preferably, an identical or similar amount for the at least one metabolite in the test sample and the reference is indicative for a subject being at risk for recurrent prostate carcinoma after prostatectomy whereas an amount in the test sample which in comparison to the reference differs is indicative for a subject being not at risk for recurrent prostate carcinoma after prostatectomy.

In another preferred embodiment of the aforementioned method of the invention, said reference is derived from a subject or group of subjects known not to be at risk for recurrent prostate carcinoma after prostatectomy. More preferably, an identical or similar amount for the at least one metabolite in the test sample and the reference is indicative for a subject being not at risk for recurrent prostate carcinoma after prostatectomy whereas an amount in the test sample which in comparison to the reference differs is indicative for a subject being at risk for recurrent prostate carcinoma after prostatectomy.

The comparison is, preferably, assisted by automation. For example, a suitable computer program comprising algorithm for the comparison of two different data sets (e.g., data sets comprising the values of the characteristic feature(s)) may be used. Such computer programs and algorithm are well known in the art. Notwithstanding the above, a comparison can also be carried out manually.

The aforementioned methods for the determination of the at least one metabolite can be implemented into a device. A device as used herein shall comprise at least the aforementioned means. Moreover, the device, preferably, further comprises means for comparison and evaluation of the detected characteristic feature(s) of the at least one metabolite and, also preferably, the determined signal intensity. The means of the device are, preferably, operatively linked to each other. How to link the means in an operating manner will depend on the type of means included into the device. For example, where means for automatically qualitatively or quantitatively determining the metabolite or metabolites are applied, the data obtained by said automatically operating means can be processed by, e.g., a computer program in order to facilitate the diagnosis. Preferably, the means are comprised by a single device in such a case. Said device may accordingly include an analyzing unit for the metabolites and an evaluation unit for processing the resulting data for the diagnosis. Alternatively, where means such as test stripes are used for determining the metabolites, the means for diagnosing may comprise control stripes or tables allocating the determined result data to result data known to be accompanied with prostate carcinoma or a predisposition therefor or those being indicative for a healthy subject as discussed above. Preferred devices are those which can be applied without the particular knowledge of a specialized clinician, e.g., test stripes or electronic devices which merely require loading with a sample.

Alternatively, the methods for the determination of the at least one metabolite can be implemented into a system comprising several devices which are, preferably, operatively linked to each other. Specifically, the means must be linked in a manner as to allow carrying out the method of the present invention as described in detail above. Therefore, operatively linked, as used herein, preferably, means functionally linked. Depending on the means to be used for the system of the present invention, said means may be functionally linked by connecting each mean with the other by means which allow data transport in between said means, e.g., glass fibre cables, and other cables for high throughput data transport. Nevertheless, wireless data transfer between the means is also envisaged by the present invention, e.g., via LAN (Wireless LAN, W-LAN). A preferred system comprises means for determining metabolites. Means for determining metabolites as used herein, encompass means for separating metabolites, such as chromatographic devices, and means for metabolite determination, such as mass spectrometry devices. Suitable devices have been described in detail above. Preferred means for compound separation to be used in the system of the present invention include chromatographic devices, more preferably devices for liquid chromatography, HPLC, and/or gas chromatography. Preferred devices for compound determination comprise mass spectrometry devices, more preferably, GC-MS, LC-MS, direct infusion mass spectrometry, FT-ICR-MS, CE-MS, HPLC-MS, quadrupole mass spectrometry, sequentially coupled mass spectrometry (including MS-MS or MS-MS-MS), ICP-MS, Py-MS or TOF. The separation and determination means are, preferably, coupled to each other. Most preferably, LC-MS and/or GC-MS is used in the system of the present invention as described in detail elsewhere in the specification. Further comprised shall be means for comparing and/or analyzing the results obtained from the means for determination of metabolites. The means for comparing and/or analyzing the results may comprise at least one database and an implemented computer program for comparison of the results.

As described above, in a preferred embodiment of the method of the present invention, said determining of the at least one metabolite comprises mass spectrometry (MS).
Mass spectrometry as used herein encompasses all techniques which allow for the determination of the molecular weight (i.e. the mass) or a mass variable corresponding to a compound, i.e. a metabolite, to be determined in accordance with the present invention. Preferably, mass spectrometry as used herein relates to GC-MS, LC-MS, direct infusion mass spectrometry, FT-ICR-MS, CE-MS, HPLC-MS, quadrupole mass spectrometry, any sequentially coupled mass spectrometry such as MS-MS or MS-MS-MS, ICP-MS, Py-MS, TOF or any combined approaches using the aforementioned techniques. How to apply these techniques is well known to the person skilled in the art. Moreover, suitable devices are commercially available. More preferably, mass spectrometry as used herein relates to LC-MS and/or GC-MS, i.e. to mass spectrometry being operatively linked to a prior chromatographic separation step. More preferably, mass spectrometry as used herein encompasses quadrupole MS. Most preferably, said quadrupole MS is carried out as follows: a) selection of a mass/charge quotient (m/z) of an ion created by ionisation in a first analytical quadrupole of the mass spectrometer, b) fragmentation of the ion selected in step a) by applying an acceleration voltage in an additional subsequent quadrupole which is filled with a collision gas and acts as a collision chamber, selection of a mass/charge quotient of an ion created by the fragmentation process in step b) in an additional subsequent quadrupole, whereby steps a) to c) of the method are carried out at least once and analysis of the mass/charge quotient of all the ions present in the mixture of substances as a result of the ionisation process, whereby the quadrupole is filled with collision gas but no acceleration voltage is applied during the analysis. Details on said most preferred mass spectrometry to be used in accordance with the present invention can be found in WO 03/073464.
More preferably, said mass spectrometry is liquid chromatography (LC) MS and/or gas chromatography (GC) MS.
Liquid chromatography as used herein refers to all techniques which allow for separation of compounds (i.e. metabolites) in liquid or supercritical phase. Liquid chromatography is characterized in that compounds in a mobile phase are passed through the stationary phase. When compounds pass through the stationary phase at different rates they become separated in time since each individual compound has its specific retention time (i.e. the time which is required by the compound to pass through the system). Liquid chromatography as used herein also includes HPLC. Devices for liquid chromatography are commercially available, e.g. from Agilent Technologies, USA. Gas chromatography as applied in accordance with the present invention, in principle, operates comparable to liquid chromatography. However, rather than having the compounds (i.e. metabolites) in a liquid mobile phase which is passed through the stationary phase, the compounds will be present in a gaseous volume. The compounds pass the column which may contain solid support materials as stationary phase or the walls of which may serve as or are coated with the stationary phase. Again, each compound has a specific time which is required for passing through the column. Moreover, in the case of gas chromatography it is preferably envisaged that the compounds are derivatised prior to gas chromatography. Suitable techniques for derivatisation are well known in the art. Preferably, derivatisation in accordance with the present invention relates to methoxymation and trimethylsilylation of, preferably, polar compounds and transmethylation, methoxymation and trimethylsilylation of, preferably, non-polar (i.e. lipophilic) compounds.
Advantageously, it has been found in accordance with the present invention that at least one or a group of the aforementioned metabolites will be a suitable biomarker for diagnosing the risk for recurrent prostate carcinoma in patients after prostatectomy. Applying these metabolites as biomarkers allows a rapid, reliable and cost-effective risk post-surgery stratification. Thus, health care management shall greatly benefit from the method of the present invention in that the need for monitoring measures as well as post-surgery care and therapeutic measures can be reliably assessed and estimated.
In principle, the invention encompasses the use of at least one biomarker selected from Table 2 or a detection agent therefor for diagnosing in a sample of a subject after prostatectomy whether said subject is at risk for recurrent prostate carcinoma after prostatectomy. Further, the disclosure relates to the use of at least one biomarker selected from Table 2 or a detection agent therefor for the manufacture of a diagnostic or pharmaceutical composition for diagnosing in a sample of a subject after prostatectomy whether said subject is at risk for recurrent prostate carcinoma after prostatectomy.

The explanations and interpretations of the terms made above apply accordingly to the other embodiments specified herein below except as indicated otherwise.

In a preferred embodiment of the aforementioned method, said method further comprises the step of recommending a therapeutic or patient health management measure for the subject based on whether the subject is diagnosed to be at risk for recurrent prostate carcinoma after prostatectomy or as metastasizing or non-metastasizing prostate carcinoma, preferably, based on a biopsy sample.

The term "recommending" as used herein refers to making suggestions for therapeutic measures and/or patient health management measures which are specifically applicable to the patient. Recommending does, preferably, not encompass the actual application of the recommended therapeutic or patient health management measure.

The term "therapeutic or patient health management measure" as used herein refers to therapeutic measures aiming to cure or ameliorate prostate cancer or aiming to prevent recurrent prostate cancer as well as patient health management measures such as monitoring including selection of monitoring measures and monitoring frequency and hospitalization. Preferably, the said therapeutic or patient health management measure is selected from the group consisting of: prostate surgery, administration of anti-cancer drugs, patient monitoring, active surveillance, watchful waiting, and hospitalization. Suitable prostate carcinoma therapies include surgery, low- and high-dose irradiation, hormone therapy and systemic chemotherapy, e.g., cytostatics, alone or in combination with other drugs, such as docetaxel in combination with prednisolone as frist-line.therapy, docetaxel combined with hormone therapy or with cytostatics like vinorelbine, epirubicine, capecitabine, or calcitriole. It will be understood that the method can also be applied to determine whether a subject will benefit from or is in need of a therapy against a progressing prostate carcinoma. Such a method can be applied in therapeutic approaches like "active surveillance". In this approach, a subject suffering from less advanced prostate carcinoma is subjected to a method for diagnosing progressing prostate carcinoma as set forth above on a short regular basis in order to detect the early onset of progression. Only after the progression becomes detectable, the subject will be treated by a suitable therapy, such as surgery or radiation. Thus, "active surveillance" prevents the harmful side effects of a therapy in subjects which are - although suffering prostate carcinoma - not in an immediate need for a therapy. By avoiding the therapy at this stage, it will be understood that the harmful side effects of the therapy can be avoided as well. The "active surveillance" approach is usually practiced with younger subjects (Kim S 2nd, Dall'Era MA, Evans CP Curr Opin Urol. 2012 ;22(3):247-53. Economic analysis of active surveillance for localized prostate cancer.)
The approach of "watchful waiting" is based on a hormonal therapy and monitoring by applying the methods of the present invention on a prolonged regular basis. If no signs of progressing prostate carcinoma are apparent, further therapeutic measures such as surgery or radiation and their side effects can be avoided (Dall'Era MA, Carroll PR. Curr Opin Urol. 2009 May;19(3):258-62, "Outcomes and follow -up strategies for patients on active surveillance"

In a more preferred embodiment of the method of the present invention, said method also comprises the step of applying the said therapeutic or patient health management measure as identified by the aforementioned method to the subject.
Accordingly, the present invention relates to a method for diagnosing whether a subject is at risk for recurrent prostate carcinoma after prostatectomy comprising:
(a) determining the amount of at least one metabolite selected from Table 2 or 2-Hydroxybehenic acid, Isopentenyl pyrophosphate or 7-methylguanine in a test sample of a subject prior to prostatectomy; and
(b) comparing the amount determined in step (a) to a reference, whereby it is diagnosed whether the subject is at risk for recurrent prostate carcinoma after prostatectomy, wherein said at least one metabolite comprises 7-methylguanine, and
   wherein said test sample is a biopsy taken before a planned prostatectomy or the tissue extracted during prostatectomy itself.
Preferably, the said sample has been obtained from the subject (e.g. by biopsy) prior to prostatectomy, however, at a time when the prostate cancer is already clinically apparent. More preferably, the prostate cancer in this context is clinically apparent if the criteria mentioned in the accompanying Examples as preoperative inclusion criteria are met.
If a sample obtained prior to prostatectomy is to be used, the method can also, preferably, be used for deciding whether prostatectomy shall be carried out on a patient, or not. Advantageously, it has been found in accordance with the present invention that at least one or a group of the aforementioned metabolites will be a suitable biomarker for diagnosing the risk for recurrent prostate carcinoma in patients prior to prostatectomy.
The nine metabolites to be used in accordance with the aforementioned method of the present invention belong to different metabolic classes. Four of the seven up-regulated metabolites, namely, cerebronic acid, hydroxybehenic acid, tricosanoic acid, and glycerophosphoethanolamine, are components of complex lipids. The three fatty acids give an indication of the probable cause of this alteration. For example, α-hydroxy long-chain fatty acids (hydroxybehenic acid, cerebronic acid) cannot be metabolized by β-oxidation but only by α-oxidation, which is localized in peroxisomes (Singh 1997, Mol Cell Biochem 167: 1-29). Cerebronic acid, as a major constituent of cerebrosides, is decarboxylated by α-oxidation to tricosanoic acid and CO2 (Sandhir 2000, Lipids 35:1127-33). The accumulation of these fatty acids in prostate cancer tissue indicates the involvement of peroxisomal α-oxidation in these changes (Zha 2005, Prostate 63:316-323). Increased levels of glycerophosphoethanolamine have already been described in prostate cancer samples (Swanson 2008, Magn Reson Med 60: 33-40). With regard to the other three increased metabolites, it should be noted that isopentenyl pyrophosphate as intermediate in the steroid synthesis pathway is formed from acetyl-CoA in the mevalonate pathway, and its increase reflects the increased cholesterol values in prostate cancer tissue (Lombard 2011, Mol Biol Evol 28: 87-99; Thysell 2010, PLoS One 5:e14175). Aminoadipic acid is an end product of the oxidation of lysine (Sell 2007, Biochem J 404: 269-277). Only recently, the uptake of lysine and secretion of aminoadipic acid was found to be increased in cell culture experiments with tumor suppressor KLF4 deficient cells (Bellance 2012, Biochim Biophys Acta 1817: 2060-71). The metabolism of aminoadipic acid was altered in consequence of the changed metabolism of lysine that forms acetyl-CoA for the increased fatty acid synthesis. Therefore, the authors suggested aminoadipic acid as potential biomarker in cancer indicating differential expression of KLF4 that functions as transcription factor (Bellance 2012, loc cit). In this respect, it is of interest that the expression of KLF4 in prostate cancer tissue is generally decreased and has prognostic value for predicting metastasis (Wang 2010, Cancer Res 70: 10182-91). Thus, the results of aminoadipic acid as prognosticator of biochemical recurrence according to the studies underlying the invention, the relation between aminoadipic acid and KLF4, and the literature data of the prognostic value of KLF4 in prostate cancer explain each other. 7-methylguanine is a repair product of methylation damage to DNA (Ames 1989, Mutat Res 214: 41-6). Increased methylguanine levels have also been found in other tumors. A reduced defense against intracellular reactive oxygen species, either due to enzymatic or zinc-based deficiencies, has been suggested as the cause of increased occurrence of methylguanine (Saad 2006, Cancer Epidemiol Biomarkers Prev 15: 740-3; Newberne 1997, Patrhobiology 65: 253-63). The two downregulated metabolites, gluconic acid, and maltotriose are related to carbohydrate metabolism. Gluconic acid connects the glucose and pentose phosphate pathways, and changed concentrations have been found under conditions of oxidative stress (Liu 2010, Burns 36: 992-8). Maltotriose, which has been known until now only as an intermediate degradation product of ingested starch by the digestive enzyme amylase, was recently discovered to be synthesized in chorioncarcinoma cells exhibiting distinct immunoregulatory activities (Zhu 2011, Am J Reprod Immunol 65: 54-64). It was also shown that maltotriose represents one of the essential nuclear localization signal sequence motifs (Niikura 2008, Chembiochem 9: 2623-7). These signal molecules govern the transport of proteins between the nuclear and cytoplasmic compartments by the so-called nuclear protein import cycle in order to maintain their distinctive composition.

Applying the aforementioned metabolites according to the invention as biomarkers allows a rapid, reliable and cost-effective risk stratification prior to prostatectomy and, thus, to decide on the usefulness of prostatectomy on a patient. Accordingly, health care management shall greatly benefit from the method of the present invention in that the need for surgery measures can be better assessed and monitoring measures as well as pre- and post-surgery care and therapeutic measures can be reliably estimated.

In principle, the invention encompasses the use of at least one biomarker selected from Table 2 or 2-Hydroxybehenic acid, Isopentenyl pyrophosphate or 7-methylguanine or a detection agent therefor for diagnosing in a sample of a subject prior to prostatectomy whether said subject is at risk for recurrent prostate carcinoma after prostatectomy. Further, the invention relates to the use of at least one biomarker selected from Table 2 or 2-Hydroxybehenic acid, Isopentenyl pyrophosphate or 7-methylguanine or a detection agent therefor for the manufacture of a diagnostic or pharmaceutical composition for diagnosing in a sample of a subject prior to prostatectomy whether said subject is at risk for recurrent prostate carcinoma after prostatectomy.

In a preferred embodiment of the aforementioned method, said method further comprises the step of recommending a therapeutic or patient health management measure for the subject based on whether the subject is diagnosed to be at risk for recurrent prostate carcinoma after prostatectomy.
In a preferred embodiment, the present invention relates to a method for diagnosing whether a subject is at risk for recurrent prostate carcinoma after prostatectomy comprising:
(a) determining the amount of at least one metabolite selected from Table 7 and/ or Table 8 and/or Table 2, in a test sample of a subject prior to prostatectomy; and
(b) comparing the amount determined in step (a) to a reference, whereby it is diagnosed whether the subject is at risk for recurrent prostate carcinoma after prostatectomy, wherein said at least one metabolite comprises 7-methylguanine, and wherein said test sample is a biopsy taken before a planned prostatectomy or the tissue extracted during prostatectomy itself.
Preferably, the term "test sample of a subject prior to prostatectomy" relates to a sample obtained before the prostata of a subject or a part thereof has been removed surgically. Accordingly, preferably, a test sample may be a biopsy sample taken before a planned prostatectomy or the tissue extracted during prostatectomy itself. More preferably, the test sample is a biopsy sample taken before a planned prostatectomy.
In a preferred embodiment, diagnosing whether a subject is at risk for recurrent prostate carcinoma after prostatectomy relates to diagnosing whether said subject will benefit from prostatectomy. Accordingly, in a preferred embodiment, diagnosing whether a subject is at risk for recurrent prostate carcinoma after prostatectomy relates to providing an aid in deciding whether prostatectomy will be beneficial for said subject, or not. Preferably, diagnosing whether a subject is at risk for recurrent prostate carcinoma after prostatectomy relates to establishing a prognosis of said subject after prostatectomy, i.e., more preferably, estimating the risk of said patient of having a poor prognosis after prostatectomy.
As will be appreciated by the skilled person, preferably, the methods for diagnosing whether a subject is at risk for recurrent prostate carcinoma allow for an improved prediction of said risk. Accordingly, the number of participants in a study, e.g. for the identification of factors increasing or decreasing said risk may be reduced. Thus, in a preferred embodiment, a method for diagnosing whether a subject is at risk for recurrent prostate carcinoma according to the present invention is part of a method for detecting factors increasing or decreasing the risk for recurrent prostate carcinoma. Preferably, factors increasing the risk for recurrent prostate carcinoma are environmental or socioeconomic factors including, without limitation, toxins, carcinogens, nutrition, and the like. Also preferably, factors decreasing the risk for recurrent prostate carcinoma include, without limitation, medical treatments or socioeconomic factors like , e.g., cytostatic treatment, radiation therapy, nutrition, and the like.
In a further preferred embodiment, a method for diagnosing whether a subject is at risk for re-current prostate carcinoma according to the present invention is part of a method for decreasing the number of subjects required for detecting a factor increasing or decreasing said risk. Moreover, in an also preferred embodiment, a method for diagnosing whether a subject is at risk for recurrent prostate carcinoma according to the present invention is part of a method for identifying a subject suitable as a test subject in a method for identifying factors increasing or decreasing said risk. Conversely, in an also preferred embodiment, a method for diagnosing whether a subject is at risk for recurrent prostate carcinoma according to the present invention is part of a method for identifying a subject not suitable as a test subject in a method for identifying factors increasing or decreasing said risk.

Moreover, the disclosure relates to a method for differentiating between a malignant prostate carcinoma tissue and a non-malignant tissue comprising:
(a) determining the amount of at least one metabolite selected from Table 4 in a test tissue sample of a subject suffering from prostate carcinoma; and
(b) comparing the amount determined in step (a) to a reference, whereby it is differentiated between malignant and non-malignant prostate tissue such that the tissue is identified as being malignant prostate carcinoma tissue or non-malignant tissue.
The term "differentiating" as used herein relates to identifying a test tissue sample as either being malignant prostate carcinoma tissue or as non-malignant and, preferably, normal prostate tissue. It will be understood that the differentiation may not be correct for all investigated tissue samples. However, preferably it is envisaged that the identification can be made for statistically significant number of cases. Whether a number of cases are statistically significant can be determined by statistical techniques described elsewhere herein.
The term "malignant prostate carcinoma tissue" refers to prostate tissue which comprises malignant prostate carcinoma cells.
The term "non-malignant tissue" refers to prostate tissue which comprises no malignant prostate carcinoma cells.

In a preferred embodiment of the aforementioned method, said reference is derived from a known malignant prostate carcinoma tissue or several known malignant prostate tissues. Preferably, an identical or similar amount for the at least one metabolite in the test tissue sample and the reference is indicative for a malignant prostate carcinoma tissue whereas an amount in the test tissue sample which in comparison to the reference differs is indicative for a non-malignant tissue.

In another preferred embodiment of the aforementioned method, said reference is derived from a known non-malignant prostate tissue. Preferably, an identical or similar amount for the at least one metabolite in the test tissue sample and the reference is indicative for a non-malignant tissue whereas an amount in the test tissue sample which in comparison to the reference differs is indicative for a malignant prostate carcinoma tissue.

In a preferred embodiment of the aforementioned method, said method further comprises the step of recommending a therapeutic or patient health management measure for the subject based on whether the test tissue sample of the subject is identified as malignant prostate carcinoma tissue or non-malignant tissue.

Advantageously, it has been found in the studies underlying the present disclosure that the aforementioned metabolites of Table 4 are reliable and efficient discriminators for malignant prostate carcinoma tissue versus non-malignant tissue in the same individual. Thus, the invention may assist in deciding on the extent of prostatectomy during surgery measures or may be used during high-throughput screening approaches.

In principle, the disclosure also encompasses the use of at least one metabolite selected from Table 4 or a detection agent therefor for differentiating in a tissue sample of a subject suffering from prostate carcinoma between a malignant prostate carcinoma tissue and a non-malignant tissue. Further encompassed is the use of at least one metabolite selected from Table 4 or a detection agent therefor for the manufacture of a diagnostic or pharmaceutical composition for differentiating in a tissue sample of a subject suffering from prostate carcinoma between a malignant prostate carcinoma tissue and a non-malignant tissue.

Also, the present disclosure relates to a method for diagnosing metastasizing prostate carcinoma comprising
(a) determining the amount of at least one metabolite in a test sample of a subject suffering from said prostate carcinoma, wherein said metabolite is selected from the group consisting of: aminoadipic acid, cerebronic acid, 2-hydroxybehenic acid, isopentenyl pyrophosphate, 7-methylguanine, tricosanoic acid and glycerophosphoethanolamine, polar fraction; and
(b) comparing the amount determined in step (a) to a reference, whereby metastasizing prostate carcinoma is diagnosed.

In the context of the present disclosure marker glycerophosphoethanolamine is, preferably, determined in the polar fraction.
The term "metastasizing prostate carcinoma" refers to advanced stage prostate carcinoma having cells capable of forming metastases outside the prostate gland. Usually, metastases from prostate carcinomas will preferentially be expected in lymph nodes or the bones.

In a preferred embodiment of the aforementioned method, said reference is derived from a subject or group of subjects known to suffer from metastasizing prostate carcinoma. Preferably, an identical or similar amount for the at least one metabolite in the test sample and the reference is indicative for metastasizing prostate carcinoma whereas an amount in the test tissue sample which in comparison to the reference differs is indicative for a non-metastasizing prostate carcinoma.

In another preferred embodiment of the method of the aforementioned method, said reference is derived from a subject or group of subjects known not to suffer from metastasizing prostate carcinoma or is a calculated reference. Preferably, an identical or similar amount for the at least one metabolite in the test sample and the reference is indicative for non-metastasizing prostate carcinoma whereas an amount in the test sample which in comparison to the reference differs is indicative for a metastasizing prostate carcinoma.

In a preferred embodiment of the aforementioned method, said method further comprises the step of recommending a therapeutic or patient health management measure for the subject based on whether the subject is diagnosed as metastasizing or non-metastasizing prostate carcinoma.

Advantageously, it has been found in the studies underlying the present disclosure that patients showing a particular high amount for any one of the aforementioned specific metabolites shall suffer from metastasizing prostate carcinoma. Thanks to the present disclosure, it is possible to identify those subjects and to apply special therapeutic or patent health management measures to them. For example, those patients shall be more closely investigated for metastases in, e.g., the bones and lymph nodes.

The present disclosure, in principle, also relates to the use of at least one biomarker or a detection agent therefor for diagnosing metastasizing prostate carcinoma in a sample of a subject suspected to suffer therefrom, wherein said at least one metabolite is selected from the group consisting of: aminoadipic acid, cerebronic acid, 2-hydroxybehenic acid, isopentenyl pyrophosphate, 7-methylguanine, tricosanoic acid and glycerophosphoethanolamine (preferably, determined in the polar fraction). Further encompassed is the use of at least one biomarker or a detection agent therefor for the manufacture of a diagnostic or pharmaceutical composition for diagnosing metastasizing prostate carcinoma in a sample of a subject suspected to suffer therefrom, wherein said at least one metabolite is selected from the group consisting of: : aminoadipic acid, cerebronic acid, 2-hydroxybehenic acid, isopentenyl pyrophosphate, 7-methylguanine, tricosanoic acid and glycerophosphoethanolamine (preferably determined in the polar fraction).

The present disclosure provides a device for diagnosing whether a subject is at risk for recurrent prostate carcinoma after prostatectomy comprising:
a) an analysing unit comprising a detection agent for at least one metabolite selected from Table 2 or 2-Hydroxybehenic acid, Isopentenyl pyrophosphate or 7-methylguanine, preferably, arranged with a detector such that the amount of the said at least one metabolite in a tissue sample can be determined; and
b) an evaluation unit comprising a data processor and a database with a stored reference, preferably, as defined above, wherein the evaluation unit has tangibly embedded an algorithm which carries out a comparison as defined above between the determined amount for the at least one metabolite received from the analysing unit and the stored reference.
A device as used herein shall comprise at least the aforementioned units. The units of the device are operatively linked to each other. How to link the means in an operating manner will depend on the type of units included into the device. For example, where the detector allows for automatic qualitative or quantitative determination of the biomarker, the data obtained by said automatically operating analyzing unit can be processed by, e.g., a computer program in order to facilitate the assessment in the evaluation unit. Preferably, the units are comprised by a single device in such a case. Said device may accordingly include an analyzing unit for the biomarker and a computer or data processing device as evaluation unit for processing the resulting data for the assessment and for stabling the output information. Preferred devices are those which can be applied without the particular knowledge of a specialized clinician, e.g., electronic devices which merely require loading with a sample. The output information of the device, preferably, is a numerical value which allows drawing conclusions on the presence or absence of a disease or condition and, thus, is an aid for diagnosis. More preferably, the output information is a preliminary diagnosis or an aid for diagnosis based on the aforementioned numerical value, i.e. a classifier which indicates whether the subject shows a disease or condition, or not. Such a preliminary diagnosis may need the evaluation of further information which can be provided in the device of the invention by including an expert knowledge database system.

A preferred reference to be used as a stored reference in accordance with the device of the present invention is an amount derived from a subject or group of subjects known to be at risk for recurrent prostate carcinoma after prostatectomy. In such a case, the algorithm tangibly embedded, preferably, compares the determined amount for the at least one biomarker with the reference, wherein an identical or similar amount for the at least one metabolite in the test sample and the reference is indicative for a subject being at risk for recurrent prostate carcinoma after prostatectomy whereas an amount in the test sample which in comparison to the reference differs is indicative for a subject being not at risk for recurrent prostate carcinoma after prostatectomy.

Another preferred reference to be used as a stored reference in accordance with the device of the present invention is an amount derived from a subject or group of subjects known not to be at risk for recurrent prostate carcinoma after prostatectomy. In such a case, the algorithm tangibly embedded, preferably, compares the determined amount for the at least one biomarker with the reference, wherein an identical or similar amount for the at least one metabolite in the test sample and the reference is indicative for a subject being not at risk for recurrent prostate carcinoma after prostatectomy whereas an amount in the test sample which in comparison to the reference differs is indicative for a subject being at risk for recurrent prostate carcinoma after prostatectomy.

It will be understood that the aforementioned device can be used for samples which have been obtained prior or after to prostatectomy. If a sample obtained prior to prostatectomy is to be analysed, preferably, the reference is an amount derived from a subject or group of subjects as specified above which has also been found prior to prostatectomy in said subject or group of subjects. If a sample obtained after to prostatectomy is to be analysed, preferably, the reference is an amount derived from a subject or group of subjects as specified above which has also been found after prostatectomy in said subject or group of subjects.

If a sample obtained prior to prostatectomy is to be used, the device can also be used for deciding whether prostatectomy shall be carried out on a patient, or not.

The units of the device, also preferably, can be implemented into a system comprising several devices which are operatively linked to each other. Depending on the units to be used for the system of the present invention, said means may be functionally linked by connecting each mean with the other by means which allow data transport in between said means, e.g., glass fibre cables, and other cables for high throughput data transport. Nevertheless, wireless data transfer between the means is also envisaged by the present invention, e.g., via LAN (Wireless LAN, W-LAN). A preferred system comprises means for determining biomarkers. Means for determining biomarkers as used herein encompass means for separating biomarkers, such as chromatographic devices, and means for metabolite determination, such as mass spectrometry devices. Suitable devices have been described in detail above. Preferred means for compound separation to be used in the system of the present invention include chromatographic devices, more preferably devices for liquid chromatography, HPLC, and/or gas chromatography. Preferred devices for compound determination comprise mass spectrometry devices, more preferably, GC-MS, LC-MS, direct infusion mass spectrometry, FT-ICR-MS, CE-MS, HPLC-MS, quadrupole mass spectrometry, sequentially coupled mass spectrometry (including MS-MS or MS-MS-MS), ICP-MS, Py-MS or TOF. The separation and determination means are, preferably, coupled to each other. Most preferably, LC-MS and/or GC-MS are used in the system of the present invention as described in detail elsewhere in the specification. Further comprised shall be means for comparing and/or analyzing the results obtained from the means for determination of biomarkers. The means for comparing and/or analyzing the results may comprise at least one databases and an implemented computer program for comparison of the results. Preferred embodiments of the aforementioned systems and devices are also described in detail below.
The disclosure further provides a device for differentiating between a malignant prostate carcinoma tissue and a non-malignant tissue comprising:
a) an analysing unit comprising a detection agent for at least one metabolite selected from Table 4, preferably, arranged with a detector such that the amount of the said at least one metabolite in a tissue sample can be determined; and
b) an evaluation unit comprising a data processor and a database with a stored reference, preferably, as defined above, wherein the evaluation unit has tangibly embedded an algorithm which carries out a comparison as defined above between the determined amount for the at least one metabolite received from the analysing unit and the stored reference.
A preferred reference to be used as a stored reference in accordance with the device of the present invention is an amount derived from a known non-malignant tissue. In such a case, the algorithm tangibly embedded, preferably, compares the determined amount for the at least one biomarker with the reference, wherein an identical or similar amount for the at least one metabolite in the test tissue sample and the reference is indicative for a non-malignant tissue whereas an amount in the test tissue sample which in comparison to the reference differs is indicative for a malignant prostate carcinoma tissue.
Another preferred reference to be used as a stored reference in accordance with the device of the present disclosure is an amount derived from a known malignant prostate carcinoma tissue.

In such a case, the algorithm tangibly embedded, preferably, compares the determined amount for the at least one biomarker with the reference, wherein an identical or similar amount for the at least one metabolite in the test tissue sample and the reference is indicative for a malignant prostate carcinoma tissue whereas an amount in the test tissue sample which in comparison to the reference differs is indicative for a non-malignant tissue
Furthermore provided is a device for diagnosing metastasizing prostate carcinoma comprising:
a) an analysing unit comprising a detection agent for at least one metabolite selected from the group consisting of: aminoadipic acid, cerebronic acid, 2-hydroxybehenic acid, isopentenyl pyrophosphate, 7-methylguanine, tricosanoic acid and glycerophosphoethanolamine, preferably determined in the polar fraction, arranged with a detector such that the amount of the said at least one metabolite in a tissue sample can be determined; and
b) an evaluation unit comprising a data processor and a database with a stored reference, preferably, as defined elsewhere herein, wherein the evaluation unit has tangibly embedded an algorithm which carries out a comparison according as set forth elsewhere herein between the determined amount for the at least one metabolite received from the analysing unit and the stored reference.
A preferred reference to be used as a stored reference in accordance with the device of the present disclosure is an amount derived from a subject or group of subjects known to suffer from metastasizing prostate carcinoma. In such a case, the algorithm tangibly embedded, preferably, compares the determined amount for the at least one biomarker with the reference, wherein an identical or similar amount for the at least one metabolite in the test sample and the reference is indicative for metastasizing prostate carcinoma whereas an amount in the test tissue sample which in comparison to the reference differs is indicative for a non-metastasizing prostate carcinoma.
Another preferred reference to be used as a stored reference in accordance with the device of the present invention is an amount derived from a subject or group of subjects known not to suffer from metastasizing prostate carcinoma or is a calculated reference. In such a case, the algorithm tangibly embedded, preferably, compares the determined amount for the at least one biomarker with the reference, wherein an identical or similar amount for the at least one metabolite in the test sample and the reference is indicative for non-metastasizing prostate carcinoma whereas an amount in the test sample which in comparison to the reference differs is indicative for a metastasizing prostate carcinoma.
In the following, some further embodiments of the invention including also preferred embodiments of the above are detailed.

The present disclosure in general, relates to a data collection comprising characteristic values of at least one metabolite being indicative for a disease or condition referred to herein.

The term "data collection" refers to a collection of data which may be physically and/or logically grouped together. Accordingly, the data collection may be implemented in a single data storage medium or in physically separated data storage media being operatively linked to each other. Preferably, the data collection is implemented by means of a database. Thus, a database as used herein comprises the data collection on a suitable storage medium. Moreover, the database, preferably, further comprises a database management system. The database management system is, preferably, a network-based, hierarchical or object-oriented database management system. Furthermore, the database may be a federal or integrated database. More preferably, the database will be implemented as a distributed (federal) system, e.g. as a Client-Server-System. More preferably, the database is structured as to allow a search algorithm to compare a test data set with the data sets comprised by the data collection. Specifically, by using such an algorithm, the database can be searched for similar or identical data sets being indicative for prostate carcinoma or a predisposition thereof (e.g. a query search). Thus, if an identical or similar data set can be identified in the data collection, the test data set will be associated with prostate carcinomas or a predisposition therefor or the progression of prostate carcinoma. Consequently, the information obtained from the data collection can be used to diagnose prostate carcinomas or a predisposition therefore based on a test data set obtained from a subject. More preferably, the data collection comprises characteristic values of all metabolites comprised by any one of the groups recited above.

In light of the foregoing, the present disclosure encompasses a data storage medium comprising the aforementioned data collection.
The term "data storage medium" as used herein encompasses data storage media which are based on single physical entities such as a CD, a CD-ROM, a hard disk, optical storage media, or a diskette. Moreover, the term further includes data storage media consisting of physically separated entities which are operatively linked to each other in a manner as to provide the aforementioned data collection, preferably, in a suitable way for a query search.

The present disclosure also relates to a system comprising:
(a) means for comparing characteristic values of metabolites of a sample operatively linked to
(b) a data storage medium as described above.
The term "system" as used herein relates to different means which are operatively linked to each other. Said means may be implemented in a single device or may be physically separated devices which are operatively linked to each other. The means for comparing characteristic values of metabolites operate, preferably, based on an algorithm for comparison as mentioned before. The data storage medium, preferably, comprises the aforementioned data collection or database, wherein each of the stored data sets being indicative for prostate carcinomas or a predisposition therefor. Thus, the system of the present disclosure allows identifying whether a test data set is comprised by the data collection stored in the data storage medium. Consequently, the system of the present disclosure may be applied as a diagnostic means in diagnosing prostate carcinomas or a predisposition or progression therefor.

In a preferred embodiment of the system, means for determining characteristic values of metabolites of a sample are comprised.
The term "means for determining characteristic values of metabolites" preferably relates to the aforementioned devices for the determination of metabolites such as mass spectrometry devices, NMR devices or devices for carrying out chemical or biological assays for the metabolites.

Moreover, the present disclosure relates to a diagnostic means comprising means for the determination of at least one metabolite selected from any one of the groups referred to above. The term "diagnostic means", preferably, relates to a diagnostic device, system or biological or chemical assay as specified elsewhere in the description in detail.
The expression "means for the determination of at least one metabolite" refers to devices or agents which are capable of specifically recognizing the metabolite. Suitable devices may be spectrometric devices such as mass spectrometry, NMR devices or devices for carrying out chemical or biological assays for the metabolites. Suitable agents may be compounds which specifically detect the metabolites. Detection as used herein may be a two-step process, i.e. the compound may first bind specifically to the metabolite to be detected and subsequently generate a detectable signal, e.g., fluorescent signals, chemiluminescent signals, radioactive signals and the like. For the generation of the detectable signal further compounds may be required which are all comprised by the term "means for determination of the at least one metabolite". Compounds which specifically bind to the metabolite are described elsewhere in the specification in detail and include, preferably, enzymes, antibodies, ligands, receptors or other biological molecules or chemicals which specifically bind to the metabolites. In a preferred embodiment the detectable signal also represent a quantifiable signal, meaning the relative intensity of the at least one metabolite is proportional to the relative intensity of the detectable signal.

Further, the present disclosure relates to a diagnostic composition comprising at least one metabolite selected from any one of the groups referred to above.

Further, the present disclosure relates to a diagnostic composition comprising at least one detection agent for at least one metabolite selected from any one of the groups referred to above. The at least one metabolite selected from any of the aforementioned groups will serve as a biomarker, i.e. an indicator molecule for a pathological condition or predisposition in the subject, i.e. prostate carcinomas or a predisposition therefor. Thus, the metabolites itself may serve as diagnostic compositions, preferably, upon visualization or detection by the means referred to in herein. Thus, a diagnostic composition which indicates the presence of a metabolite according to the present invention may also comprise the said biomarker physically, e.g., a complex of an antibody and the metabolite to be detected may serve as the diagnostic composition. Accordingly, the diagnostic composition may further comprise means for detection of the metabolites as specified elsewhere in this description. Alternatively, if detection means such as MS or NMR based techniques are used, the molecular species which serves as an indicator for the pathological condition will be the at least one metabolite comprised by the test sample to be investigated. Thus, the at least one metabolite referred to in accordance with the present disclosure shall serve itself as a diagnostic composition due to its identification as a biomarker.

### FIGURES

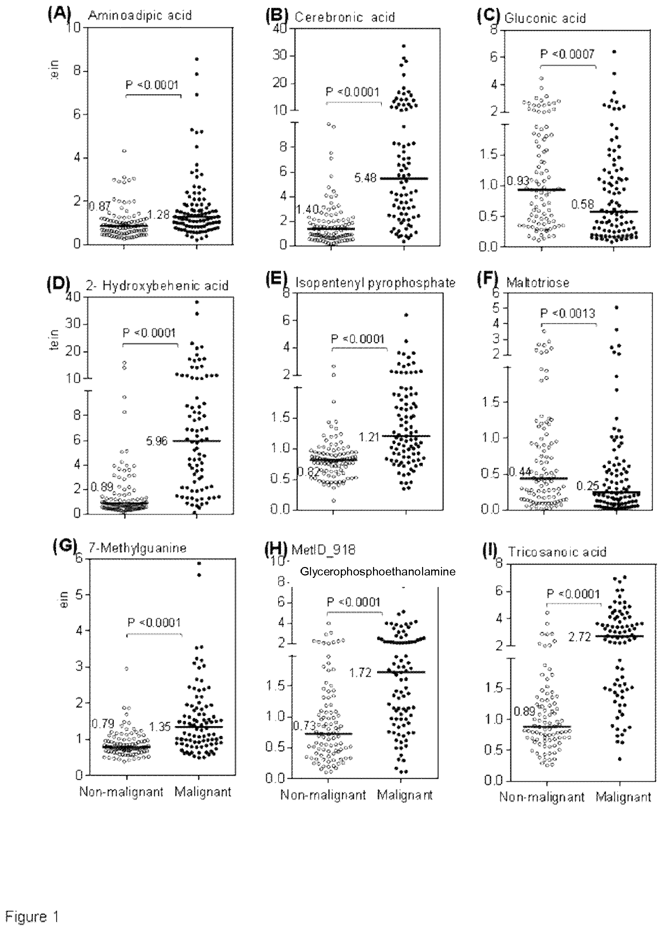

Fig. 1 shows metabolites in matched malignant and non-malignant prostatectomy samples (y-axis: relative amount of the metabolite normalized to a reference pool). Differences were tested by the Wilcoxon matched pairs test. Because of missing values as mentioned in the text, there were between 83 and 93 complete matched pairs of samples from the total cohort of 95 patients. The paired samples actually evaluated for each metabolite are indicated in the respective figure in brackets.
Fig. 2 shows Kaplan-Meier curves for biochemical recurrence-free time after surgery by (A) pathological tumor stage, (B) Gleason score, and levels of (C) aminoadipic acid, (D) gluconic acid, (E) maltotriose (F) Glycerophosphoethanolamine, (G) Tricosanoic acid and (H) Crebronic Acid. The stratification of the metabolites corresponded to the data given in Table 5.

### EXAMPLES

The invention will now be illustrated by the following Examples which are not intended to restrict or limit the scope of this invention.

### Example 1: Determination of metabolites

### Patients and tissue samples

Tumor tissue and matched normal adjacent tissue were taken from prostate specimens immediately cryopreserved in liquid nitrogen after radical prostatectomy between 2001 and 2007. For this retrospective study, the selection of samples from 95 men with untreated prostate carcinoma before surgery was made according to the availability of cryo-preserved tissues and the completeness of follow-up data on the basis of sample size calculation as mentioned below. The study was approved by the local ethical board and informed patient consent was obtained. Methodical details to obtain pure tumor tissue (>90%) and matched adjacent normal tissue were described previously (see WO2010/139711). For each patient, clinicopathological information on age, prostate volume, preoperative PSA, tumor classification according to the UICC 2002 TNM System, tumor Gleason grade based on the whole specimen, and PSA concentrations during postoperative follow-up were compiled (Table 1).

### Measurement of metabolites and PSA

All the metabolites were measured in the framework of a global metabolite profiling study by gas chromatography-mass spectrometry and liquid chromatography-mass spectrometry. Samples collected as described in the previous chapter were prepared and subjected to metabolite profiling by gas chromatography-mass spectrometry (GC-MS) and liquid chromatography-mass spectrometry (LC-MS/MS) analysis. The freeze-dried tissue material was extracted with polar (water) and non-polar (ethanol/dichloromethane/acetonitrile) solvents. The extract was fractioned into an aqueous, polar phase (Polar fraction) and an organic, lipophilic phase (lipid fraction). For GC-MS analyses, the lipid fraction was treated with methanol under acidic conditions to yield the fatty acid methyl esters derived from both free fatty acids and hydrolyzed complex lipids. Further, components of the lipid backbone (i.e. glycerol) were quantified in the non-polar phase (carrying the term "lipid fraction" following the metabolite name). For example, "glycerol, lipid fraction" represents glycerol liberated from complex lipids - in contrast, "glycerol, polar fraction" represents glycerol present originally in the polar phase of the biological sample. The polar and lipid fractions were further derivatized with O-methyl-hydroxylamine hydrochloride (20 mg/ml in pyridine, 50 µl) to convert oxo-groups to O-methyloximes and subsequently with a silylating agent (MSTFA, 50 µl) before GC-MS. For LC-MS/MS analyses, both fractions were reconstituted in appropriate solvent mixtures. High performance liquid chromatography (HPLC) was performed by gradient elution using methanol/water/formic acid on reversed phase separation columns. Mass spectrometric detection technology was applied as described in the patent US 7196323, which allows targeted and high sensitivity "Multiple Reaction Monitoring" profiling in parallel to a full screen analysis. Tissue samples were analyzed in semi-randomized analytical sequence design (samples of each subject analyzed in subsequent slots, subjects and sequence of tissue randomized) with pooled samples (= "pool") generated from extra samples provided for this purpose. The raw peak data were normalized to the median of pool per analytical sequence to account for process variability (so called "ratios versus pool"). This calculated arbitrary unit was related to the tissue weight and resulted in the specification arbitrary unit/mg tissue wet weight. A rigorous quality control was performed on peak, analyte and sample level. Within each analytical sequence, the signals of the internal standards were plotted onto control charts. Samples that displayed >30% standard deviation of one of the internal standards, were invalidated. Outlier peaks on group level (carcinoma tissue, control tissue) were identified by boxplot analysis, manually checked for correct annotation and integration and, if necessary, manually corrected. Peaks with very low metabolite abundance that did not allow reliable peak integration or that did not meet requirements of retention time index were converted to missing values. This procedure explains the different missing values for the various analytes in the 95 samples evaluated in this study. Metabolite values were normalized to the weight of each sample as well as to the median of reference samples derived from a pool generated from separate prostate tissue samples. Thus, all metabolite data were presented as arbitrary units related to these reference samples.

Determinations of PSA were performed on an Elecsys analyzer (Roche Diagnostics, Mannheim, Germany).

### Outcome evaluation

The diagnostic validities of metabolites were evaluated regarding their capability to discriminate between tumor and normal tissue. Receiver-operating characteristics (ROC) analysis and binary logistic regression were applied, and the areas under the curves (AUC), sensitivity, specificity, and total correct classification rates were calculated. The prognostic potential of the metabolites was assessed to predict tumor recurrence after surgery. The primary end point was recurrence-free survival, as measured from the date of surgery to the last follow-up or the first postoperative PSA value of >0.2 ng/ml, confirmed by subsequent rising values following an undetectable PSA level (<0.04 ng/ml) after surgery.

### Statistical analyses

The GraphPad Prism 6.0 (GraphPad Software, San Diego, CA, USA), SPSS Statistics 19 (IBM, Chicago, IL, USA), and MedCalc 12.3.0 (MedCalc Software, Mariakerke, Belgium) software packages were used. The Mann-Whitney U-test, Wilcoxon matched pairs test, Spearman rank correlation, Chi-square test, Kaplan-Meier analyses, univariate and multivariate Cox regressions, and ROC analyses were performed. The SPSS bootstrapping modul was used. The "missing value" modul of SPSS was used to analyze the structure of missing values using Little's MCAR test.

### Example 2: Evaluation of the biomarkers in patients

### Patient characteristics

Ninety-five PCa patients were investigated in this study (Table 1). Within a median follow-up of 52 months, 74 men were recurrence free and 21 suffered tumor relapse. There were a few missing values of metabolites due to technical reasons (Table 2). Little's MCAR test proved that the missing values were completely at random (p=0.140). This condition allows computations with a list-wise deletion of data or application of single imputation methods to replace missing values. We used both approaches and mention them at the appropriate places.

**Table 1: Clinical characteristics of the study group.^{a}**

| Characteristics | Total | No recurrence | Recurrence | *p* value |
|---|---|---|---|---|
| Number | 95 | 74 | 21 | |
| Age, years | | | | 0.559^{b} |
| Median (95% CI) | 64 (62.5-65) | 64 (62.1-65) | 64 (61.6-65) | |
| Range | 46-73 | 46-73 | 46-71 | |
| Preoperative PSA, ng/ml | | | | 0.701^{b} |
| Median (95% CI) | 7.5 (6.7-8.6) | 7.5 (6.5-9.0) | 7.5 (5.9-12.4) | |
| Range | 1.7-41.9 | 1.8-41.9 | 1.7-34.9 | |
| Preoperative %fPSA | | | | 0.472^{b} |
| Median (95% CI) | 8.6 (7.4-10.0) | 8.2 (7.0-10.3) | 9.1 (7.3-13.9) | |
| Range | 2.8-45.3 | 2.8-45.3 | 3.2-18.3 | |
| Prostate volume, cm³ | | | | 0.879^{b} |
| Median (95% CI) | 32 (30-35) | 32 (28-35) | 35 (25-40) | |
| Range | 13-98 | 15-98 | 13-92 | |
| No. pathological stage (%) | | | | <0.0003^{c} |
| pT2a | 4 (4) | 4 (5) | | |
| pT2b | 15 (16 | 12 (16) | 3 (14) | |
| pT2c | 42 (44 | 40 (54 | 2 (10) | |
| pT3a | 28 (29) | 15 (20) | 13 (62) | |
| pT3b | 6 (6) | 3(4) | 3 (14) | |
| No. Gleason score (%) | | | | <0.0001^{c} |
| 5 | 6 (6) | 6 (8) | - | |
| 6 | 24 (25) | 23 (31) | 1 (5) | |
| 7 | 41 (43) | 35 (47) | 6 (28 | |
| 8 | 13 (14) | 5 (7) | 8 (38) | |
| 9 | 10 (11) | 5(7) | 5(24) | |
| 10 | 1 (1) | - | 1 (5) | |
| Follow-up, mo | | | | <0.0001^{b} |
| Median (95% CI) | 52.1 (46.1-56.8) | 55.5 (50.4-66.2) | 23.4 (10.9-42.3) | |
| Range | 0.6-114 | 8.3-114 | 0.6-105.2 | |

| | | | | |
|---|---|---|---|---|
| CI: confidence interval; PSA: prostate-specific antigen; %fPSA: percentage ratio of free to total PSA. ^{a} Values are given as medians with 95% CI in parentheses and ranges or numbers of patients with percentages in parentheses. ^{b} Significances between no-recurrence and recurrence tested by Mann-Whitney *U*-test. ^{c} Significances between no-recurrence and recurrence tested by Chi-square test. | | | | |

**Table 2: Available metabolite data in the subgroups of patients without and with recurrence following radical prostatectomy**

| | Number of patients with available metabolite data | | |
|---|---|---|---|
| Metabolite | Total group | Patients without recurrence | Patients with recurrence |
| | n=95 (100%) | n=74 (100%) | n=21 (100%) |
| **Aminoadipic acid** | 93 (97.9) | 72 (97.3) | 21 (100) |

| | n=95 (100%) | n=74 (100%) | n=21 (100%) |
|---|---|---|---|
| Cerebronic acid | 87 (91.6) | 68 (91.9) | 19 (90.5) |
| **Gluconic acid** | 92 (96.8) | 72 (97.3) | 20 (95.2) |
| Glycerophosphoethanolamine | 93 (97.9) | 72 (97.3) | 21 (100) |
| **Maltotriose** | 92 (96.8) | 71 (95.9) | 21 (100) |
| Tricosanoic acid | 86 (90.5) | 67 (90.5) | 19 (90.5) |

### Metabolites in malignant and non-malignant prostate tissue

The global metabolite profiling study was established on 107 matched-paired tissue samples. A large number of 822 analytes were detected; 566 were classified "qualitative" and 256 (161 known, 95 unknown) could be quantified. Twelve patients with incomplete follow-ups of this initial profiling study were excluded from the subsequent data evaluation. For nine of the top metabolites belonging to different metabolic classes, namely aminoadipic acid, cerebronic acid, gluconic acid, glycerophospho-ethanolamine, 2-hydroxybehenic acid, isopentenyl pyrophosphate, maltotriose, 7-methylguanine, and tricosanoic acid significant differences were found between non-malignant and malignant tissue samples, with higher concentrations in tumor samples except for gluconic acid and maltotriose, which had lower levels in tumors (Figure 1). However, no significant differences in the metabolites dependent on Gleason score (<7 vs. ≥7; p=0.089 to 0.979) or tumor stage (pT2 vs. pT3; p=0.150 to 0.887) except for gluconic acid (p=0.021; Table 3) were observed. Age, preoperative PSA, prostate volume, tumor stage, and Gleason score were not significantly correlated (p>0.05) with any of the metabolites in the tumor samples.

**Table 3A: Metabolites in carcinoma samples depending on the Gleason score.^{a}**

| Variable | Patients with Gleason score <7^{b} | Patients with Gleason score ≥7^{b} | *p* value^{c} |
|---|---|---|---|
| Aminoadipic acid | 1.20 (0.61 - 7.87) n=28 | 1.34 (0.22 - 8.56) n=65 | 0.802 |
| Cerebronic acid | 3.35 (0.65 - 18.3) n=26 | 6.14 (0.36 - 33.8) n=61 | 0.089 |
| Gluconic acid | 0.81 (0.12 - 4.84) n=28 | 0.49 (0.09 - 6.44) n=64 | 0.157 |
| Glycerophosphoethanolamine | 1.68 (0.17 - 3.99) n=28 | 1.73 (0.11 - 9.31) n=65 | 0.782 |
| 2-Hydroxybehenic acid | 4.45 (0.68 - 17.2) n=24 | 6.78 (0.14 - 38.2) n=59 | 0.178 |
| Isopentenyl pyrophosphate | 1.22 (0.35 - 2.65) n=30 | 1.18 (0.37 - 6.41) n=62 | 0.934 |
| Maltotriose | 0.31 (0.04 - 5.08) n=28 | 0.21 (0.01 - 3.64) n=64 | 0.338 |
| 7-Methylguanine | 1.47 (0.51 - 3.04) n=27 | 1.30 (0.50 - 5.88) n=62 | 0.979 |
| Tricosanoic acid | 2.35 (0.76 - 5.65) n=25 | 2.99 (0.36 - 7.06) n=61 | 0.534 |

| | | | |
|---|---|---|---|
| ^{a} Metabolite results are presented as medians (arbitrary units) with 95 CI in parentheses. ^{b} Number of patients with available metabolite data are indicated by n in the corresponding row. ^{c} Significances were tested by the Mann-Whitney *U* test. | | | |

**Table 3B: Metabolites in carcinoma samples depending on the pathological stage.^{a}**

| Variable | Patients with pT2^{b} | Patients with pT3^{b} | *p* value^{c} |
|---|---|---|---|
| Aminoadipic acid | 1.23 (0.22 - 7.87) n=58 | 1.44 (0.29 - 8.56) n=35 | 0.247 |
| Cerebronic acid | 4.28 (0.36 - 33.8) n=52 | 6.14 (0.87 - 29.3) n=35 | 0.174 |
| Gluconic acid | 0.81 (0.09 - 4.84) n=58 | 0.38 (0.12 - 6.44) n=34 | 0.021 |
| Glycerophosphoethanolamine | 1.70 (0.12 - 5.13) n=58 | 1.75 (0.11 - 9.31) n=35 | 0.887 |
| 2 - Hydroxybehenic acid | 5.06 (0.14 - 38.2) n=50 | 6.85 (0.91 - 34.0) n=33 | 0.434 |
| Isopentenyl pyrophosphate | 1.11 (0.35 - 4.49) n=57 | 1.34 (0.37 - 6.41) n=35 | 0.269 |
| Maltotriose | 0.29 (0.02 - 5.08) n=58 | 0.20 (0.01 - 1.86) n=34 | 0.150 |
| 7 - Methylguanine | 1.32 (0.50 - 5.56) n=55 | 1.41 (0.61 - 5.88) n=34 | 0.303 |
| Tricosanoic acid | 2.65 (0.36 - 7.06) n=52 | 3.28 (0.63 - 6.71) n=34 | 0.268 |

| | | | |
|---|---|---|---|
| ^{a} Metabolite results are presented as medians (arbitrary units) with 95% CI in parentheses. ^{b} Number of patients with available metabolite data are indicated by n in the corresponding row. ^{c} Significances were tested by the Mann-Whitney *U* test. | | | |

### Discriminative potential of metabolites between malignant and non-malignant tissue

AUCs of the ROC analyses and correct classification rates determined, in correspondence with the data in Figure 1A-H, the discrimination performance of all metabolites to distinguish malignant from non-malignant tissue (Table 4). Six metabolites showed AUCs greater than 0.75.

**Table 4: Discrimination performance of metabolites between malignant and non-malignant tissue samples from prostate cancer.**

| Metabolite^{a} | AUC (95% CI)^{b} | Correct classification (%) | | |
|---|---|---|---|---|
| | | Non-malignant | Malignant | Overall |
| Aminoadipic acid | 0.69 (0.62 - 0.77)*** | 80 | 50 | 65 |
| Cerebronic acid | 0.84 (0.78 - 0.90)*** | 89 | 63 | 76 |
| Gluconic acid | 0.61 (0.53 - 0.69)** | 51 | 63 | 57 |
| Glycerophosphoethanol-amine | 0.76 (0.69 - 0.83)*** | 75 | 61 | 68 |
| 2-Hydroxybehenic acid | 0.85 (0.80 - 0.91)*** | 86 | 66 | 76 |
| Isopentenyl pyrophosphate | 0.76 (0.70 - 0.83)*** | 84 | 60 | 72 |
| Maltotriose | 0.66 (0.52 - 0.81)* | 40 | 76 | 58 |
| 7-Methylguanine | 0.77 (0.69 - 0.84)*** | 79 | 59 | 69 |
| Tricosanoic acid | 0.86 (0.81 - 0.92)*** | 87 | 67 | 77 |
| Combinations | | | | |
| All metabolites^{c} | 0.88 (0.82 - 0.93)*** | 90 | 70 | 80 |
| Tricosanoic acid^{d} | 0.86 (0.81 - 0.92)*** | 87 | 67 | 77 |

| | | | | |
|---|---|---|---|---|
| AUC: area under the receiver operating curve; CI: confidence interval. ^{a} Analyses were performed with the data sets available for each individual metabolite as given in Table 2 and only with cases after list wise deletion of cases with incomplete data if binary logistic regression was performed. ^{b} Significantly different from AUC = 0.5: **p*<0.05; ***p*<0.001; ****p*<0.0001. ^{c} Predicted by full logistic regression. ^{d} Predicted by stepwise logistic regression (forward or backward elimination; entry *p*=0.05, elimination *p*=0.10). | | | | |

### Prognostic potential of metabolites predicting tumor recurrence

The different metabolite profiles led us to evaluate their potential for predicting tumor recurrence defined as biochemical recurrence following radical prostatectomy. First, Kaplan-Meier curves were calculated (Figure 2A-H). The recurrence-free interval was significantly shorter with increasing tumor stage and Gleason score; this association proved that our study group was representative (Figure 2A,B). Kaplan-Meier analyses were also significant for aminoadipic acid, gluconic acid, and maltotriose (Figure 2C-E, *p*=0.004-0.030) and for tricosanoic acid, glycerophosphoethanolamine and Cerebronic acid (Figure 2F-H, *p*=0.079-0.168), while the other three metabolites were not associated with biochemical relapse (*p*=0.252-0.806). Next, all clinico-pathological factors and metabolites were assessed regarding their prognostic information in Cox regressions (Table 5). The hazard ratios of the metabolites in univariate Cox regression analyses reflected the results of the Kaplan-Meier curves. Then, both the significant pathological factors (tumor stage, Gleason score) and metabolites (aminoadipic acid, gluconic acid, and maltriose) from the univariate analyses (Table 5) were evaluated in multivariate Cox regression analyses using full and reduced models

**Table 5: Univariate and multivariate Cox proportional hazard regression analyses of tumor stage, Gleason score, and tissue metabolites in prostate cancer patients with regard to the recurrence-free interval after radical prostatectomy.**

| Variable^{a} | Hazard ratio (95% CI) | *p* |
|---|---|---|
| Univariate analysis^{b} | | |
| Tumor stage (pT2 / pT3) | 8.20 (2.77-24.3) | 0.0002 |
| Gleason score (5+6/7/8+9+10) | 4.63 (2.21-9.65) | 0.0001 |
| Age (<65/≥65 years) | 0.82 (0.34-1.96) | 0.652 |
| Prostate-specific antigen (<11.7≥ ng/ml) | 1.64 (0.65-4.10) | 0.297 |
| Aminoadipic acid (<1.85≥); n=93 | 2.58 (1.10-6.09) | 0.031 |
| Cerebronic acid (<5.27≥); n=87 | 1.98 (0.75-5.21) | 0.168 |
| Gluconic acid (<0.403≥); n=92 | 0.38 (0.16-0.94) | 0.036 |
| Glycerophosphoethanolamine (<1.15≥); n=93 | 2.20 (0.74-6.51) | 0.157 |
| 2-Hydroxybehenic acid (<5.79≥); n=83 | 1.44 (0.57-3.66) | 0.441 |
| Isopentenyl pyrophosphate (<1.21≥); n=92 | 1.11 (0.47-2.63) | 0.806 |
| Maltotriose (<0.129≥), n=92 | 0.29 (0.13-0.71) | 0.006 |
| 7-Methylguanine (<1.00≥); n=89 | 1.80 (0.66-4.88) | 0.252 |
| Tricosanoic acid (<4.14≥); n=86 | 2.31 (0.91-5.85) | 0.079 |

| Multivariate analysis, full model^{c} | | |
|---|---|---|
| Tumor stage | 3.99 (1.10-14.5) | 0.036 |
| Gleason score | 2.25 (0.93-5.40) | 0.071 |
| Aminoadipic acid | 2.37 (0.93-6.02) | 0.071 |
| Gluconic acid | 0.81 (0.28-2.35) | 0.704 |
| Maltotriose | 0.62 (0.21-1.81) | 0.384 |

| | | |
|---|---|---|
| ^{a} The categorization criteria are given in brackets. Metabolites were dichotomized using their values (given in arbitrary units) obtained in the ROC analysis as the point of maximal accuracy to discriminate between recurrence/non-recurrence. ^{b} Univariate analyses using the metabolites were done with the available data for the respective metabolite indicated by "n" (see also Table 2) while for the other variables complete data for all 95 cases were available. ^{c} Full model with significant individual variables from the univariate analyses (p <0.05). The approach of excluding cases with missing values list wise was used with the result that the analysis was performed with complete data set of 92 patients as indicated in Table 2. | | |

**Table 6: Univariate and multivariate Cox proportional hazard regression analyses using cases with only complete data and with missing values replaced by the regression based imputation of values.^{a}**

| Variable^{b} | Analysis of cases with complete data only Hazard ratio (95% CI) | *p* | Analysis with missingvalues replaced by imputation Hazard ratio (95% CI) | *p* |
|---|---|---|---|---|
| Univariate analysis^{c} | | | | |
| Aminoadipic acid (<1.85≥); n=93 | 2.58 (1.10-6.09) | 0.031 | 3.06 (1.30-7.22) | 0.011 |
| Cerebronic acid (<5.27≥); n=87 | 1.98 (0.75-5.21) | 0.168 | 1.95 (0.79-4.83) | 0.150 |
| Gluconic acid (<0.403≥); n=92 | 0.38 (0.16-0.94) | 0.036 | 0.39 (0.16-0.93) | 0.034 |
| Glycerophosphoethanolamine (<1.15≥); n=93 | 2.20 (0.74-6.51) | 0.157 | 2.32 (0.79-6.90) | 0.129 |
| 2-Hydroxybehenic acid (<5.79≥); n=83 | 1.44 (0.57-3.66) | 0.441 | 1.55 (0.64-3.74) | 0.330 |
| Isopentenyl pyrophosphate (<1.21≥); n=92 | 1.11 (0.47-2.63) | 0.806 | 1.05 (0.44-2.46) | 0.918 |
| Maltotriose (<0.129≥), n=92 | 0.29 (0.13-0.71) | 0.006 | 0.31 (0.13-0.74) | 0.009 |
| 7-Methylguanine (<1.00≥); n=89 | 1.80 (0.66-4.88) | 0.252 | 1.94 (0.72-5.28) | 0.195 |
| Tricosanoic acid (<4.14≥); n=86 | 2.31 (0.91-5.85) | 0.079 | 2.14 (0.87-5.29) | 0.100 |

| Multivariate analysis, full model^{d} | | | | |
|---|---|---|---|---|
| Tumor stage | 3.99 (1.10-14.5) | 0.036 | 3.65 (0.97-13.7) | 0.056 |
| Gleason score | 2.25 (0.93-5.40) | 0.071 | 2.14 (0.89-5.17) | 0.092 |
| Aminoadipic acid | 2.37 (0.93-6.02) | 0.071 | 2.26 (0.92-5.68) | 0.077 |
| Gluconic acid | 0.81 (0.28-2.35) | 0.704 | 0.96 (0.32-2.91) | 0.943 |
| Maltotriose | 0.62 (0.21-1.81) | | 0.384 0.70 (0.23-2.17) | 0.705 |

| | | | | |
|---|---|---|---|---|
| CI: confidence interval. ^{a} Missing values were replaced by imputed values using the regression-residual-based procedure in the MVA modul of SPPS 19. ^{b} The categorization criteria are given in brackets. Metabolites were dichotomized using their values (given in arbitrary units) obtained in the ROC analysis as the point of maximal accuracy to discriminate between recurrence/non-recurrence. ^{c} Univariate analyses were done with the available data for the respective metabolite indicated by "n" (see also Table 2). ^{d} Full model with significant individual variables from the univariate analyses (p <0.05). The approach of excluding cases with missing values list wise was used with the result that the analysis was performed with a complete data set of 92 patients as indicated in Table 2. | | | | |

**Table 7: Metabolites useful in methods for diagnosing whether a subject is at risk for recurrent prostate carcinoma**

| METABOLITE_NAME | **Median of carcinoma tissue relative to control** | **p-value of paired t-test, two-sided** | **AUC carcinoma versus healthy tissue** |
|---|---|---|---|
| 7-methylguanine | 1,55 | 0,0000 | 0,75 |
| 2-Hydroxybehenic acid | 6,62 | 0,0000 | 0,85 |
| Isopentenyl pyrophosphate | 1,45 | 0,0000 | 0,75 |

**Table 8: Further metabolites useful in methods for diagnosing whether a subject is at risk for recurrent prostate carcinoma**

| METABOLITE_NAME | **Median of carcinoma tissue relative to control** | **p-value of paired t-test, two-sided** | **AUC carcinoma versus healthy tissue** |
|---|---|---|---|
| Cerebronic acid (2-OH-C24:0) | 3,73 | 0,0000 | 0,82 |
| 14-Methylhexadecanoic acid | 1,14 | 0,0000 | 0,62 |
| 2-Aminoadipinic acid | 1,45 | 0,0000 | 0,68 |
| Ceramide (d18:1, C24:1) | 1,10 | 0,0011 | 0,60 |
| Eicosenoic acid (C20:cis[11]1) | 1,55 | 0,0000 | 0,70 |
| Tricosanoic acid (C23:0) | 2,99 | 0,0000 | 0,85 |
| Eicosadienoic acid (C20:2) No 02 | 1,53 | 0,0000 | 0,72 |
| Arginine | 1,18 | 0,0000 | 0,62 |
| Behenic acid (C22:0) | 1,37 | 0,0000 | 0,69 |
| beta-Carotene | 1,25 | 0,0000 | 0,60 |
| Cholestenol No 02 | 1,33 | 0,0000 | 0,69 |
| Cytosine | 1,14 | 0,0000 | 0,66 |
| DAG (C18:1, C18:2) | 1,04 | 0,0020 | 0,56 |
| Dihydrocholesterol | 1,35 | 0,0000 | 0,66 |
| erythro-Dihydrosphingosine | 1,12 | 0,0056 | 0,58 |
| Docosahexaenoic acid (C22:cis[4,7,10,13,16,19]6) | 1,24 | 0,0000 | 0,65 |
| Dodecanol | 1,08 | 0,0323 | 0,57 |
| Eicosanoic acid (C20:0) | 1,41 | 0,0000 | 0,68 |
| Eicosapentaenoic acid (C20:cis[5,8,11,14,17]5) | 1,20 | 0,0003 | 0,60 |
| Eicosatrienoic acid (C20:3) | 1,20 | 0,0000 | 0,64 |
| erythro-C16-Sphingosine | 1,45 | 0,0000 | 0,69 |
| Flavine adenine dinucleotide (FAD) | 1,35 | 0,0000 | 0,69 |
| gamma-Tocopherol | 1,37 | 0,0000 | 0,67 |
| Gluconic acid | 0,59 | 0,0002 | 0,62 |
| Glucuronic acid | 0,67 | 0,0001 | 0,62 |
| Glycerol-2-phosphate | 1,48 | 0,0000 | 0,74 |
| Lignoceric acid (C24:0) | 1,33 | 0,0000 | 0,68 |
| Lysophosphatidylcholine (C18:2) | 1,16 | 0,0000 | 0,62 |
| Lysophosphatidylcholine (C20:4) | 1,06 | 0,0007 | 0,57 |
| Maltotriose | 0,54 | 0,0000 | 0,62 |
| myo-Inositol, lipid fraction | 1,13 | 0,0007 | 0,63 |
| myo-Inositol-2-phosphate, lipid fraction (myo-Inositolphospholipids) | 1,44 | 0,0000 | 0,70 |
| Nervonic acid (C24:cis[15]1) | 1,15 | 0,0002 | 0,62 |
| Nicotinamide | 1,13 | 0,0004 | 0,61 |
| Pentadecanol | 1,52 | 0,0000 | 0,75 |
| Phosphatidylcholine (C18:0, C22:6) | 0,88 | 0,0004 | 0,62 |
| Phytosphingosine | 1,24 | 0,0002 | 0,64 |
| Pseudouridine | 1,10 | 0,0005 | 0,62 |
| Pyruvate | 1,07 | 0,0004 | 0,60 |
| 3-O-Methylsphingosine | 1,33 | 0,0001 | 0,67 |
| threo-Sphingosine | 1,33 | 0,0001 | 0,68 |
| 5-O-Methylsphingosine | 1,30 | 0,0002 | 0,66 |
| erythro-Sphingosine | 1,32 | 0,0012 | 0,64 |
| Sphingosine-1-phosphate | 1,26 | 0,0005 | 0,65 |
| Threonic acid | 1,17 | 0,0011 | 0,60 |
| Homoserine | 1,16 | 0,0000 | 0,66 |
| Hypoxanthine | 1,71 | 0,0000 | 0,76 |
| Lysine | 1,29 | 0,0000 | 0,70 |
| Oleic acid (C18:cis[9]1) | 1,33 | 0,0000 | 0,69 |

**Table 9: Chemical/physical properties of selected metabolites from Table 8.**

| METABOLITE_NAME | **Characteristics** |
|---|---|
| | 5-O-Methylsphingosine exhibits the following characteristic ionic fragments when detected with GC/MS, applying electron impact (EI) ionization mass spectrometry, after acidic methanolysis and derivatisation with 2% O-methylhydroxylamine-hydrochlorid in pyridine and subsequently with N-methyl-N-trimethylsilyltrifluoracetamid: |
| 5-O-Methylsphingosine | MS (EI, 70 eV): m/z (%): 250 (100), 73 (34), 251 (19), 354 (14), 355 (4), 442 (1). |
| | Cholestenol No 02 represents a Cholestenol isomer. It exhibits the following characteristic ionic fragments when detected with GC/MS, applying electron impact (EI) ionization mass spectrometry, after acidic methanolysis and derivatisation with 2% O-methylhydroxylamine-hydrochlorid in pyridine and subsequently with N-methyl-N-trimethylsilyltrifluoracetamid: |
| Cholestenol No 02 | MS (EI, 70 eV): m/z (%): 143 (100), 458 (91), 73 (68), 81 (62), 95 (36), 185 (23), 327 (23), 368 (20), 255 (15), 429 (15). |
| | 3-O-Methylsphingosine exhibits the following characteristic ionic fragments when detected with GC/MS, applying electron impact (EI) ionization mass spectrometry, after acidic methanolysis and derivatisation with 2% O-methylhydroxylamine-hydrochlorid in pyridine and subsequently with N-methyl-N-trimethylsilyltrifluoracetamid: |
| 3-O-Methylsphingosine | MS (EI, 70 eV): m/z (%): 204 (100), 73 (18), 205 (16), 206 (7), 354 (4), 442 (1). |
| | Eicosadienoic acid (C20:2) No 02 represents an Eicosadienoic acid isomer. It exhibits the following characteristic ionic fragments when detected with GC/MS, applying electron impact (EI) ionization mass spectrometry, after acidic methanolysis and derivatisation with 2% O-methylhydroxylamine-hydrochlorid in pyridine and subsequently with N-methyl-N-trimethylsilyltrifluoracetamid: |
| Eicosadienoic acid (C20:2) No 02 | MS (EI, 70 eV): m/z (%): 81 (100), 57 (98), 43 (92), 67 (85), 41 (80), 55 (74), 82 (66), 95 (64), 110 (39), 109 (39). |

## Claims

1. An in vitro method for diagnosing whether a subject is at risk for recurrent prostate carcinoma after prostatectomy comprising:
(a) determining the amount of at least one metabolite selected from the list consisting of 7-methylguanine, 2-Hydroxybehenic acid, Isopentenyl pyrophosphate, Cerebronic acid (2-OH-C24:0), 14-Methylhexadecanoic acid, 2-Aminoadipinic acid, Ceramide (d18:1, C24:1), Eicosenoic acid (C20:cis[11]1), Tricosanoic acid (C23:0), Eicosadienoic acid (C20:2) No 02, Arginine, Behenic acid (C22:0), beta-Carotene, Cholestenol No 02, Cytosine , DAG (C18:1, C18:2), Dihydrocholesterol, erythro-Dihydrosphingosine, Docosahexaenoic acid (C22:cis[4,7,10,13,16,19]6), Dodecanol, Eicosanoic acid (C20:0), Eicosapentaenoic acid (C20:cis[5,8,11,14,17]5), Eicosatrienoic acid (C20:3), erythro-C16-Sphingosine, Flavine adenine dinucleotide (FAD), gamma-Tocopherol, Gluconic acid, Glucuronic acid, Glycerol-2-phosphate, Lignoceric acid (C24:0), Lysophosphatidylcholine (C18:2), Lysophosphatidylcholine (C20:4), Maltotriose, myo-Inositol (lipid fraction), myo-Inositol-2-phosphate (lipid fraction, myo-Inositolphospholipids), Nervonic acid (C24:cis[15]1), Nicotinamide, Pentadecanol, Phosphatidylcholine (C18:0, C22:6), Phytosphingosine, Pseudouridine, Pyruvate , 3-O-Methylsphingosine, threo-Sphingosine, 5-O-Methylsphingosine, erythro-Sphingosine, Sphingosine-1-phosphate, Threonic acid, Homoserine, Hypoxanthine , Lysine, Oleic acid (C18:cis[9]1), and Glycerophosphoethanolamine in a test sample of a subject prior to prostatectomy; and
(b) comparing the amount determined in step (a) to a reference, whereby it is diagnosed whether the subject is at risk for recurrent prostate carcinoma after prostatectomy, wherein said at least one metabolite comprises 7-methylguanine, and
wherein said test sample is a biopsy sample taken before a planned prostatectomy or the tissue extracted during prostatectomy itself.

2. The method of claim 1, wherein said reference is derived from a subject or group of subjects known to be at risk for recurrent prostate carcinoma after prostatectomy, preferably wherein an identical or similar amount for the at least one metabolite in the test sample and the reference is indicative for a subject being at risk for recurrent prostate carcinoma after prostatectomy whereas an amount in the test sample which in comparison to the reference differs is indicative for a subject being not at risk for recurrent prostate carcinoma after prostatectomy.

3. The method of claim 1, wherein said reference is derived from a subject or group of subjects known not to be at risk for recurrent prostate carcinoma after prostatectomy, preferably
wherein an identical or similar amount for the at least one metabolite in the test sample and the reference is indicative for a subject being not at risk for recurrent prostate carcinoma after prostatectomy whereas an amount in the test sample which in comparison to the reference differs is indicative for a subject being at risk for recurrent prostate carcinoma after prostatectomy.

4. The method of any one of claims 1 to 3, wherein said method further comprises the step of recommending a therapeutic or patient health management measure for the subject based on whether the subject is diagnosed as being at risk for recurrent prostate carcinoma after prostatectomy.

5. A device comprising an analysing unit and an evaluation unit
(i) for diagnosing whether a subject is at risk for recurrent prostate carcinoma after prostatectomy,
a) wherein said analysing unit comprises a detection agent for at least one metabolite selected from the list consisting of 7-methylguanine, 2-Hydroxybehenic acid, Isopentenyl pyrophosphate, Cerebronic acid (2-OH-C24:0), 14-Methylhexadecanoic acid, 2-Aminoadipinic acid, Ceramide (d18:1, C24:1), Eicosenoic acid (C20:cis[11]1), Tricosanoic acid (C23:0), Eicosadienoic acid (C20:2) No 02, Arginine, Behenic acid (C22:0), beta-Carotene, Cholestenol No 02, Cytosine , DAG (C18:1, C18:2), Dihydrocholesterol, erythro-Dihydrosphingosine, Docosahexaenoic acid (C22:cis[4,7,10,13,16,19]6), Dodecanol, Eicosanoic acid (C20:0), Eicosapentaenoic acid (C20:cis[5,8,11,14,17]5), Eicosatrienoic acid (C20:3), erythro-C16-Sphingosine, Flavine adenine dinucleotide (FAD), gamma-Tocopherol, Gluconic acid, Glucuronic acid, Glycerol-2-phosphate, Lignoceric acid (C24:0), Lysophosphatidylcholine (C18:2), Lysophosphatidylcholine (C20:4), Maltotriose, myo-Inositol (lipid fraction), myo-Inositol-2-phosphate (lipid fraction, myo-Inositolphospholipids), Nervonic acid (C24:cis[15]1), Nicotinamide, Pentadecanol, Phosphatidylcholine (C18:0, C22:6), Phytosphingosine, Pseudouridine, Pyruvate , 3-O-Methylsphingosine, threo-Sphingosine, 5-O-Methylsphingosine, erythro-Sphingosine, Sphingosine-1-phosphate, Threonic acid, Homoserine, Hypoxanthine , Lysine, Oleic acid (C18:cis[9]1), and Glycerophosphoethanolamine preferably, arranged with a detector such that the amount of the said at least one metabolite in a tissue sample can be determined; and
b) wherein said evaluation unit comprises a data processor and a database with a stored reference as defined in claim 2 or 3, wherein the evaluation unit has tangibly embedded an algorithm which carries out a comparison according to claim 2 or 3 between the determined amount for the at least one metabolite received from the analysing unit and the stored reference,
wherein said at least one metabolite comprises 7-methylguanine.

6. In vitro use of at least one biomarker comprising 7-methylguanine or a detection agent therefor for diagnosing in a sample of a subject after or prior to prostatectomy whether said subject is at risk for recurrent prostate carcinoma after prostatectomy.

7. An in vitro method for diagnosing whether a subject is at risk for recurrent prostate carcinoma after prostatectomy comprising:
(a) determining the amount of at least one metabolite selected from the list consisting of 7-methylguanine, 2-Hydroxybehenic acid, Isopentenyl pyrophosphate, Cerebronic acid (2-OH-C24:0), 14-Methylhexadecanoic acid, 2-Aminoadipinic acid, Ceramide (d18:1, C24:1), Eicosenoic acid (C20:cis[11]1), Tricosanoic acid (C23:0), Eicosadienoic acid (C20:2) No 02, Arginine, Behenic acid (C22:0), beta-Carotene, Cholestenol No 02, Cytosine , DAG (C18:1, C18:2), Dihydrocholesterol, erythro-Dihydrosphingosine, Docosahexaenoic acid (C22:cis[4,7,10,13,16,19]6), Dodecanol, Eicosanoic acid (C20:0), Eicosapentaenoic acid (C20:cis[5,8,11,14,17]5), Eicosatrienoic acid (C20:3), erythro-C16-Sphingosine, Flavine adenine dinucleotide (FAD), gamma-Tocopherol, Gluconic acid, Glucuronic acid, Glycerol-2-phosphate, Lignoceric acid (C24:0), Lysophosphatidylcholine (C18:2), Lysophosphatidylcholine (C20:4), Maltotriose, myo-Inositol (lipid fraction), myo-Inositol-2-phosphate (lipid fraction, myo-Inositolphospholipids), Nervonic acid (C24:cis[15]1), Nicotinamide, Pentadecanol, Phosphatidylcholine (C18:0, C22:6), Phytosphingosine, Pseudouridine, Pyruvate , 3-O-Methylsphingosine, threo-Sphingosine, 5-O-Methylsphingosine, erythro-Sphingosine, Sphingosine-1-phosphate, Threonic acid, Homoserine, Hypoxanthine , Lysine, Oleic acid (C18:cis[9]1), and Glycerophosphoethanolamine in a test sample of a subject after prostatectomy; and
(b) comparing the amount determined in step (a) to a reference, whereby it is diagnosed whether the subject is at risk for recurrent prostate carcinoma after prostatectomy, wherein said at least one metabolite comprises 7-methylguanine, and
wherein said test sample is a sample of prostate tissue suspected to include or essentially consist of prostate carcinoma cells.

8. The method of claim 7,
(i) wherein said reference is derived from a subject or group of sub-jects known to be at risk for recurrent prostate carcinoma after prostatectomy, preferably wherein an identical or similar amount for the at least one metabolite in the test sample and the reference is indicative for a subject being at risk for recurrent prostate carcinoma after prostatectomy whereas an amount in the test sample which in comparison to the reference differs is indicative for a subject being not at risk for recurrent prostate carcinoma after prostatectomy;
or
(ii) wherein said reference is derived from a subject or group of sub-jects known not to be at risk for recurrent prostate carcinoma after prostatectomy, preferably wherein an identical or similar amount for the at least one metabolite in the test sample and the reference is indicative for a subject being not at risk for re-current prostate carcinoma after prostatectomy whereas an amount in the test sample which in comparison to the reference differs is indicative for a subject being at risk for re-current prostate carcinoma after prostatectomy.

## Patentansprüche

1. In-vitro-Verfahren zum Diagnostizieren, ob ein Individuum einem Risiko für rezidivierendes Prostatakarzinom nach Prostatektomie unterliegt, umfassend:
(a) Bestimmen der Menge von mindestens einem Metaboliten, ausgewählt aus der Liste bestehend aus 7-Methylguanin, 2-Hydroxybehensäure, Isopentenylpyrophosphat, Cerebronsäure (2-OH-C24:0), 14-Methylhexadecansäure, 2-Aminoadipinsäure, Ceramid (d18:1, C24:1), Eicosensäure (C20:cis[11]1), Tricosansäure (C23:0), Eicosadiensäure (C20:2) Nr. 02, Arginin, Behensäure (C22:0), Beta-Carotin, Cholestenol Nr. 02, Cytosin, DAG (C18:1, C18:2), Dihydrocholesterin, Erythro-Dihydrosphingosin, Docosahexaensäure (C22:cis[4,7,10,13,16,19]6), Dodecanol, Eicosansäure (C20:0), Eicosapentaensäure (C20:cis[5,8,11,14,17]5), Eicosatriensäure (C20:3), Erythro-C16-Sphingosin, Flavinadenindinucleotid (FAD), Gamma-Tocopherol, Gluconsäure, Glucuronsäure, Glycerin-2-phosphat, Lignocersäure (C24:0), Lysophosphatidylcholin (C18:2), Lysophosphatidycholin (C20:4), Maltotriose, Myo-Inositol (Lipidfraktion), Myo-Inositol-2-phosphat (Lipidfraktion, Myo-Inositolphospholipide), Nervonsäure (C24:cis[15]1), Nicotinamid, Pentadecanol, Phosphatidylcholin (C18:0, C22:6), Phytosphingosin, Pseudouridin, Pyruvat, 3-O-Methylsphingosin, Threo-Sphingosin, 5-O-Methylsphingosin, Erythro-Sphingosin, Sphingosin-1-phosphat, Threonsäure, Homoserin, Hypoxanthin, Lysin, Ölsäure (C18:cis[9]1) und Glycerophosphoethanolamin in einer Testprobe von einem Individuum vor Prostatektomie; und
(b) Vergleichen der in Schritt (a) bestimmten Menge mit einer Referenz, wobei diagnostiziert wird, ob das Individuum einem Risiko für ein rezidivierendes Prostatakarzinom nach Prostatektomie unterliegt,
wobei der mindestens eine Metabolit 7-Methylguanin umfasst, und
wobei es sich bei der Testprobe um eine Biopsieprobe, die vor einer geplanten Prostatektomie entnommen wurde, oder das Gewebe, das während der Prostatektomie selbst extrahiert wurde, handelt.

2. Verfahren nach Anspruch 1, wobei die Referenz von einem Individuum oder einer Gruppe von Individuen abgeleitet ist, von denen bekannt ist, dass sie einem Risiko für ein rezidivierendes Prostatakarzinom nach Prostatektomie unterliegen, wobei vorzugsweise eine identische oder ähnliche Menge für den mindestens einen Metaboliten in der Testprobe und der Referenz ein Individuum anzeigt, das einem Risiko für rezidivierendes Prostatakarzinom nach Prostatektomie unterliegt, wohingegen eine Menge in der Testprobe, die sich im Vergleich zur Referenz unterscheidet, ein Individuum anzeigt, das keinem Risiko für ein rezidivierendes Prostatakarzinom nach Prostatektomie unterliegt.

3. Verfahren nach Anspruch 1, wobei die Referenz von einem Individuum oder einer Gruppe von Individuen abgeleitet ist, von denen bekannt ist, dass sie keinem Risiko für ein rezidivierendes Prostatakarzinom nach Prostatektomie unterliegen, wobei vorzugsweise eine identische oder ähnliche Menge für den mindestens einen Metaboliten in der Testprobe und der Referenz ein Individuum anzeigt, das keinem Risiko für rezidivierendes Prostatakarzinom nach Prostatektomie unterliegt, wohingegen eine Menge in der Testprobe, die sich im Vergleich zu der Referenz unterscheidet, ein Individuum anzeigt, das einem Risiko für ein rezidivierendes Prostatakarzinom nach Prostatektomie unterliegt.

4. Verfahren nach einem der Ansprüche 1 bis 3, wobei das Verfahren ferner den Schritt umfasst, bei dem eine therapeutische oder Patientengesundheitsmanagementmaßnahme für das Individuum, basierend darauf empfohlen wird, ob bei dem Individuum ein Risiko für rezidivierendes Prostatakarzinom nach Prostatektomie diagnostiziert wurde.

5. Vorrichtung umfassend eine Analyseeinheit und eine Bewertungseinheit
(i) zum Diagnostizieren, ob ein Individuum einem Risiko für rezidivierendes Prostatakarzinom nach Prostatektomie unterliegt,
a) wobei die Analyseeinheit ein Nachweismittel für mindestens einen Metaboliten umfasst, der ausgewählt ist aus der Liste bestehend aus 7-Methylguanin, 2-Hydroxybehensäure, Isopentenylpyrophosphat, Cerebronsäure (2-OH-C24:0), 14-Methylhexadecansäure, 2-Aminoadipinsäure, Ceramid (d18:1, C24:1), Eicosensäure (C20:cis[11]1), Tricosansäure (C23:0), Eicosadiensäure (C20:2) Nr. 02, Arginin, Behensäure (C22:0), Beta-Carotin, Cholestenol Nr. 02, Cytosin, DAG (C18:1, C18:2), Dihydrocholesterin, Erythro-Dihydrosphingosin, Docosahexaensäure (C22:cis[4,7,10,13,16,19]6), Dodecanol, Eicosansäure (C20:0), Eicosapentaensäure (C20:cis[5,8,11,14,17]5), Eicosatriensäure (C20:3), Erythro-C16-Sphingosin, Flavinadenindinucleotid (FAD), Gamma-Tocopherol, Gluconsäure, Glucuronsäure, Glycerin-2-phosphat, Lignocersäure (C24:0), Lysophosphatidylcholin (C18:2), Lysophosphatidycholin (C20:4), Maltotriose, Myo-Inositol (Lipidfraktion), Myo-Inositol-2-phosphat (Lipidfraktion, Myo-Inositolphospholipide), Nervonsäure (C24:cis[15]1), Nicotinamid, Pentadecanol, Phosphatidylcholin (C18:0, C22:6), Phytosphingosin, Pseudouridin, Pyruvat, 3-O-Methylsphingosin, Threo-Sphingosin, 5-O-Methylsphingosin, Erythro-Sphingosin, Sphingosin-1-phosphat, Threonsäure, Homoserin, Hypoxanthin, Lysin, Ölsäure (C18:cis[9]1) und Glycerophosphoethanolamin, vorzugsweise angeordnet mit einem Detektor, so dass die Menge des mindestens einen Metaboliten in einer Gewebeprobe bestimmt werden kann; und
b) wobei die Auswerteeinheit einen Datenprozessor und eine Datenbank mit einer gespeicherten Referenz nach Anspruch 2 oder 3 umfasst, wobei in der Auswerteeinheit ein Algorithmus, der einen Vergleich nach Anspruch 2 oder 3 zwischen der bestimmten Menge für den mindestens einen Metaboliten, die von der Analyseeinheit erhalten wird, und der gespeicherten Referenz ausführt, fassbar eingebettet ist,
wobei der mindestens eine Metabolit 7-Methylguanin umfasst.

6. In-vitro-Verwendung von mindestens einem Biomarker, der 7-Methylguanosin oder ein Nachweismittel dafür umfasst, zum Diagnostizieren in einer Probe eines Individuums nach oder vor einer Prostatektomie, ob das Individuum einem Risiko für rezidivierendes Prostatakarzinom nach Prostatektomie unterliegt.

7. In-vitro-Verfahren zum Diagnostizieren, ob ein Individuum einem Risiko für rezidivierendes Prostatakarzinom nach Prostatektomie unterliegt, umfassend:
(a) Bestimmen der Menge von mindestens einem Metaboliten, ausgewählt aus der Liste bestehend aus 7-Methylguanin, 2-Hydroxybehensäure, Isopentenylpyrophosphat, Cerebronsäure (2-OH-C24:0), 14-Methylhexadecansäure, 2-Aminoadipinsäure, Ceramid (d18:1, C24:1), Eicosensäure (C20:cis[11]1), Tricosansäure (C23:0), Eicosadiensäure (C20:2) Nr. 02, Arginin, Behensäure (C22:0), Beta-Carotin, Cholestenol Nr. 02, Cytosin, DAG (C18:1, C18:2), Dihydrocholesterin, Erythro-Dihydrosphingosin, Docosahexaensäure (C22:cis[4,7,10,13,16,19]6), Dodecanol, Eicosansäure (C20:0), Eicosapentaensäure (C20:cis[5,8,11,14,17]5), Eicosatriensäure (C20:3), Erythro-C16-Sphingosin, Flavinadenindinucleotid (FAD), Gamma-Tocopherol, Gluconsäure, Glucuronsäure, Glycerin-2-phosphat, Lignocersäure (C24:0), Lysophosphatidylcholin (C18:2), Lysophosphatidycholin (C20:4), Maltotriose, Myo-Inositol (Lipidfraktion), Myo-Inositol-2-phosphat (Lipidfraktion, Myo-Inositolphospholipide), Nervonsäure (C24:cis[15]1), Nicotinamid, Pentadecanol, Phosphatidylcholin (C18:0, C22:6), Phytosphingosin, Pseudouridin, Pyruvat, 3-O-Methylsphingosin, Threo-Sphingosin, 5-O-Methylsphingosin, Erythro-Sphingosin, Sphingosin-1-phosphat, Threonsäure, Homoserin, Hypoxanthin, Lysin, Ölsäure (C18:cis[9]1) und Glycerophosphoethanolamin in einer Testprobe von einem Individuum nach Prostatektomie; und
(b) Vergleichen der in Schritt (a) bestimmten Menge mit einer Referenz, wobei diagnostiziert wird, ob das Individuum einem Risiko für ein rezidivierendes Prostatakarzinom nach Prostatektomie unterliegt,
wobei der mindestens eine Metabolit 7-Methylguanin umfasst, und
wobei die Testprobe eine Prostatagewebeprobe ist, die unter dem Verdacht steht, dass sie Prostatakarzinomzellen umfasst oder im Wesentlichen daraus besteht.

8. Verfahren nach Anspruch 7,
(i) wobei die Referenz von einem Individuum oder einer Gruppe von Individuen abgeleitet ist, von denen bekannt ist, dass sie einem Risiko für ein rezidivierendes Prostatakarzinom nach Prostatektomie unterliegen, wobei vorzugsweise eine identische oder ähnliche Menge für den mindestens einen Metaboliten in der Testprobe und der Referenz ein Individuum anzeigt, das einem Risiko für rezidivierendes Prostatakarzinom nach Prostatektomie unterliegt, wohingegen eine Menge in der Testprobe, die sich im Vergleich zur Referenz unterscheidet, ein Individuum anzeigt, das nach Prostatektomie keinem Risiko für ein rezidivierendes Prostatakarzinom unterliegt;
oder
(ii) wobei die Referenz von einem Individuum oder einer Gruppe von Individuen abgeleitet ist, von denen bekannt ist, dass sie keinem Risiko für ein rezidivierendes Prostatakarzinom nach Prostatektomie unterliegen, wobei vorzugsweise eine identische oder ähnliche Menge für den mindestens einen Metaboliten in der Testprobe und der Referenz ein Individuum anzeigt, das keinem Risiko für rezidivierendes Prostatakarzinom nach Prostatektomie unterliegt, wohingegen eine Menge in der Testprobe, die sich im Vergleich zu der Referenz unterscheidet, ein Individuum anzeigt, das einem Risiko für ein rezidivierendes Prostatakarzinom nach Prostatektomie unterliegt.

## Revendications

1. Procédé in vitro pour diagnostiquer le fait qu'un sujet est ou non à risque de carcinome de la prostate récidivant après prostatectomie comprenant :
(a) la détermination de la quantité d'au moins un métabolite choisi dans la liste constituée des 7-méthylguanine, acide 2-hydroxybéhénique, pyrophosphate d'isopentényle, acide cérébronique (2-OH-C24:0), acide 14-méthylhexadécanoïque, acide 2-aminoadipinique, céramide (d18:1,C24:1), acide eicosénoïque (C20:cis[11]1), acide tricosanoïque (C23:0), acide eicosadiénoïque (C20:2) n° 02, arginine, acide béhénique (C22:0), bêta-carotène, cholestérol n° 02, cytosine, DAG (C18:1,C18:2), dihydrocholestérol, érythro-dihydrosphingosine, acide docosahexaénoïque (C22:cis[4,7,10,13,16,19]6), dodécanol, acide eicosanoïque (C20:0), acide eicosapentaénoïque (C20:cis[5,8,11,14,17]5), acide eicosatriénoïque (C20:3), érythro-C16-sphingosine, flavine adénine dinucléotide (FAD), gamma-tocophérol, acide gluconique, acide glucuronique, glycérol-2-phosphate, acide lignocérique (C24:0), lysophosphatidylcholine (C18:2), lysophosphatidylcholine (C20:4), maltotriose, myo-inositol (fraction lipidique), myo-inositol-2-phosphate (fraction lipidique, myo-inositolphospholipides), acide nervonique (C24:cis[15]1), nicotinamide, pentadécanol, phosphatidylcholine (C18:0,C22:6), phytosphingosine, pseudouridine, pyruvate, 3-O-méthylsphingosine, thréosphingosine, 5-O-méthylsphingosine, érythrosphingosine, sphingosine-1-phosphate, acide thréonique, homosérine, hypoxanthine, lysine, acide oléique (Cl8:cis[9]1) et glycérophosphoéthanolamine dans un échantillon d'essai d'un sujet avant prostatectomie ; et
(b) comparaison de la quantité déterminée dans l'étape (a) à une référence, de sorte qu'il soit diagnostiqué que le sujet est ou non à risque de carcinome de la prostate récidivant après prostatectomie, où ledit au moins un métabolite comprend la 7-méthylguanine, et où ledit échantillon d'essai est un échantillon de biopsie prélevé avant une prostatectomie planifiée ou le tissu extrait pendant la prostatectomie elle-même.

2. Procédé de la revendication 1, dans lequel ladite référence est dérivée d'un sujet ou groupe de sujets connu pour être à risque de carcinome de la prostate récidivant après prostatectomie, de préférence dans lequel une quantité identique ou similaire pour l'au moins un métabolite dans l'échantillon d'essai et la référence est indicative qu'un sujet est à risque de carcinome de la prostate récidivant après prostatectomie tandis qu'une quantité dans l'échantillon d'essai qui, par rapport à la référence, diffère, est indicative qu'un sujet n'est pas à risque de carcinome de la prostate récidivant après prostatectomie.

3. Procédé de la revendication 1, dans lequel ladite référence est dérivée d'un sujet ou groupe de sujets connu pour ne pas être à risque de carcinome de la prostate récidivant après prostatectomie, de préférence
dans lequel une quantité identique ou similaire pour l'au moins un métabolite dans l'échantillon d'essai et la référence est indicative qu'un sujet n'est pas à risque de carcinome de la prostate récidivant après prostatectomie tandis qu'une quantité dans l'échantillon d'essai qui, par rapport à la référence, diffère, est indicative qu'un sujet est à risque de carcinome de la prostate récidivant après prostatectomie.

4. Procédé de l'une quelconque des revendications 1 à 3, où ledit procédé comprend en outre l'étape de recommandation d'une mesure thérapeutique ou de gestion de la santé de patient pour le sujet sur la base du fait que le sujet est ou non diagnostiqué comme étant à risque de carcinome de la prostate récidivant après prostatectomie.

5. Dispositif comprenant une unité d'analyse et une unité d'évaluation
(i) pour diagnostiquer le fait qu'un sujet est ou non à risque de carcinome de la prostate récidivant après prostatectomie,
a) dans lequel ladite unité d'analyse comprend un agent de détection pour au moins un métabolite choisi dans la liste constituée des 7-méthylguanine, acide 2-hydroxybéhénique, pyrophosphate d'isopentényle, acide cérébronique (2-OH-C24:0), acide 14-méthylhexadécanoïque, acide 2-aminoadipinique, céramide (d18:1,C24:1), acide eicosénoïque (C20:cis[11]1), acide tricosanoïque (C23:0), acide eicosadiénoïque (C20:2) n° 02, arginine, acide béhénique (C22:0), bêta-carotène, cholesténol n° 02, cytosine, DAG (C18:1,C18:2), dihydrocholestérol, érythro-dihydrosphingosine, acide docosahexaénoïque (C22:cis[4,7,10,13,16,19]6), dodécanol, acide eicosanoïque (C20:0), acide eicosapentaénoïque (C20:cis[5,8,11,14,17]5), acide eicosatriénoïque (C20:3), érythro-C16-sphingosine, flavine adénine dinucléotide (FAD), gamma-tocophérol, acide gluconique, acide glucuronique, glycérol-2-phosphate, acide lignocérique (C24:0), lysophosphatidylcholine (C18:2), lysophosphatidylcholine (C20:4), maltotriose, myo-inositol (fraction lipidique), myo-inositol-2-phosphate (fraction lipidique, myo-inositolphospholipides), acide nervonique (C24:cis[15]1), nicotinamide, pentadécanol, phosphatidylcholine (C18:0,C22:6), phytosphingosine, pseudouridine, pyruvate, 3-O-méthylsphingosine, thréosphingosine, 5-O-méthylsphingosine, érythrosphingosine, sphingosine-1-phosphate, acide thréonique, homosérine, hypoxanthine, lysine, acide oléique (C18:cis[9]1) et glycérophosphoéthanolamine de préférence, agencée avec un détecteur de sorte que la quantité dudit au moins un métabolite dans un échantillon de tissu puisse être déterminée ; et
b) dans lequel ladite unité d'évaluation comprend un processeur de données et une base de données avec une référence stockée telle que définie dans la revendication 2 ou 3, dans lequel l'unité d'évaluation comporte, incorporé de façon tangible, un algorithme qui effectue une comparaison selon la revendication 2 ou 3 entre la quantité déterminée pour l'au moins un métabolite reçue depuis l'unité d'analyse et la référence stockée, où ledit au moins un métabolite comprend la 7-méthylguanine.

6. Utilisation in vitro d'au moins un biomarqueur comprenant de la 7-méthylguanine ou un agent de détection pour celui-ci pour le diagnostic dans un échantillon d'un sujet après ou avant prostatectomie du fait que ledit sujet est ou non à risque de carcinome de la prostate récidivant après prostatectomie.

7. Procédé in vitro pour diagnostiquer le fait qu'un sujet est ou non à risque de carcinome de la prostate récidivant après prostatectomie comprenant :
(a) la détermination de la quantité d'au moins un métabolite choisi dans la liste constituée des 7-méthylguanine, acide 2-hydroxybéhénique, pyrophosphate d'isopentényle, acide cérébronique (2-OH-C24:0), acide 14-méthylhexadécanoïque, acide 2-aminoadipinique, céramide (d18:1,C24:1), acide eicosénoïque (C20:cis[11]1), acide tricosanoïque (C23:0), acide eicosadiénoïque (C20:2) n° 02, arginine, acide béhénique (C22:0), bêta-carotène, cholesténol n° 02, cytosine, DAG (C18:1,C18:2), dihydrocholestérol, érythro-dihydrosphingosine, acide docosahexaénoïque (C22:cis[4,7,10,13,16,19]6), dodécanol, acide eicosanoïque (C20:0), acide eicosapentaénoïque (C20:cis[5,8,11,14,17]5), acide eicosatriénoïque (C20:3), érythro-C16-sphingosine, flavine adénine dinucléotide (FAD), gamma-tocophérol, acide gluconique, acide glucuronique, glycérol-2-phosphate, acide lignocérique (C24:0), lysophosphatidylcholine (C18:2), lysophosphatidylcholine (C20:4), maltotriose, myo-inositol (fraction lipidique), myo-inositol-2-phosphate (fraction lipidique, myo-inositolphospholipides), acide nervonique (C24:cis[15]1), nicotinamide, pentadécanol, phosphatidylcholine (C18:0,C22:6), phytosphingosine, pseudouridine, pyruvate, 3-O-méthylsphingosine, thréosphingosine, 5-O-méthylsphingosine, érythrosphingosine, sphingosine-1-phosphate, acide thréonique, homosérine, hypoxanthine, lysine, acide oléique (C18:cis[9]1) et glycérophosphoéthanolamine dans un échantillon d'essai d'un sujet après prostatectomie ; et
(b) comparaison de la quantité déterminée dans l'étape (a) à une référence, de sorte qu'il soit diagnostiqué que le sujet est ou non à risque de carcinome de la prostate récidivant après prostatectomie, où ledit au moins un métabolite comprend la 7-méthylguanine, et où ledit échantillon d'essai est un échantillon de tissu de prostate suspecté de comprendre ou d'être essentiellement constitué de cellules de carcinome de la prostate.

8. Procédé de la revendication 7,
(i) dans lequel ladite référence est dérivée d'un sujet ou groupe de sujets connu pour être à risque de carcinome de la prostate récidivant après prostatectomie, de préférence dans lequel une quantité identique ou similaire pour l'au moins un métabolite dans l'échantillon d'essai et la référence est indicative qu'un sujet est à risque de carcinome de la prostate récidivant après prostatectomie tandis qu'une quantité dans l'échantillon d'essai qui, par rapport à la référence, diffère, est indicative qu'un sujet n'est pas à risque de carcinome de la prostate récidivant après prostatectomie ; ou
(ii) dans lequel ladite référence est dérivée d'un sujet ou groupe de sujets connu pour ne pas être à risque de carcinome de la prostate récidivant après prostatectomie, de préférence dans lequel une quantité identique ou similaire pour l'au moins un métabolite dans l'échantillon d'essai et la référence est indicative qu'un sujet n'est pas à risque de carcinome de la prostate récidivant après prostatectomie tandis qu'une quantité dans l'échantillon d'essai qui, par rapport à la référence, diffère est indicative qu'un sujet est à risque de carcinome de la prostate récidivant après prostatectomie.
